# EUROPEAN PATENT APPLICATION

(11) **EP 4 206 318 A1**
(43) Date of publication of application: **05.07.2023**
(21) Application number: 22198880.1
(22) Date of filing: 24.07.2013
(51) Int. Cl.: C12N 5/00, C12N 5/02, C12N 5/0735, C12N 5/074, C07K 16/28, C07K 16/30

(54) **NME VARIANT SPECIES EXPRESSION AND SUPPRESSION**

(30) Priority: 24.07.2012 US 201261675292 P; 30.07.2012 US 201261677442 P; 02.08.2012 US 201261679021 P; 14.08.2012 US 201261683155 P; 17.08.2012 US 201261684654 P; 27.08.2012 US 201261693712 P; 17.10.2012 WO PCT/US2012/060684
(62) Divisional of application: 13823099.0
(71) Applicant: Minerva Biotechnologies Corporation, Waltham, MA 02451 (US)
(72) Inventor: BAMDAD, Cynthia, Waltham, MA 02451 (US)
(74) Representative: Mewburn Ellis LLP

(57) **Abstract**

The present application discloses a method for generating less mature cells from starting cells including inducing the starting cells to revert to a less mature state including increasing the amount of an NME family member whose multimerization state is the biologically active state or decreasing the relative amount of an NME family member whose multimerization state is the biologically inactive state.

## Description

### BACKGROUND OF THE INVENTION

### 1. Field of the Invention:

The present application relates to the field of manipulating the expression of NME family proteins and their associated factors to regulate stem-like growth and treat cancer.

### 2. General Background and State of the Art

NM23 exists as a family of proteins wherein the commonality among these proteins is the presence of a nucleoside diphosphate kinase (NDPK) domain that catalyzes the conversion of ATP to ADP. NM23 has previously been known as Tumor Metastasis Factor. With the recent identification of ten NM23 family members, they are now also known as NME proteins 1-10 (Mol Cell Biochem (2009) 329:51-62, "The mammalian Nm23/NDPK family: from metastasis control to cilia movement," Mathieu Boissan , Sandrine Dabernat, Evelyne Peuchant, Uwe Schlattner, Ioan Lascu, and Marie-Lise Lacombe).

Scientists first isolated a differentiation inhibition factor from human leukemia cells and showed that the addition of this factor blocked chemically induced differentiation of certain types of leukemia and myeloid cells (Okabe-Kado, 1985, Cancer Research 45, 4848-4852, "Characterization of a Differentiation-inhibitory Activity from Nondifferentiating Mouse Myeloid Leukemia Cells); this inhibitory factor was later identified as NME1 (NM23-H1) (Okabe-Kado, 1992, "Identity of a differentiation inhibiting factor for mouse myeloid leukemia cells with NM23/nucleoside diphosphate kinase", Biochem Biophys Res Comm, 182 No.3 987-994). Leukemia cells are blood cells that are blocked from terminal differentiation. Interestingly, the ability to inhibit differentiation of leukemia cells was shown to be independent of its catalytic domain. Mutations in the NDPK domain that abrogated its enzymatic activity had no effect on the protein's ability to block differentiation of some types of leukemia cells. However, the scientific literature of the following decades paints a picture of total confusion as to whether NM23 inhibits differentiation, accelerates differentiation or has no effect at all.

Many research articles provided evidence indicating that NM23 induces differentiation. Rosengard et al, 1989, Dearolf et al 1993, and Timmons et al 1993 reported that *in vivo* the Drosophila NM23 homologue, *awd,* is required for proper differentiation. Lakso et al 1992 reported that NM23 (mouse *in vivo*) increases with initiation of tissue differentiation, implying that it induces differentiation. Yamashiro et al 1994 reported that *in vitro* NM23 levels increase during differentiation of human erythroleukemia cells. Lombardi et al 1995 concluded that NM23 (mouse *in vitro*) increases with initiation of cellular differentiation, again indicating that NM23 induces differentiation. Gervasi 1996, reported that overexpression of NM23 (rat *in vitro*) induced neuronal differentiation and down regulation of NM23 with anti-sense DNA inhibited differentiation. Amendola et al 1997 showed that transfection of NM23 in human neuroblastoma cells increased differentiation.

In direct contradiction to the many research articles that reported that NM23 induced differentiation, an equal number of papers published in the same time frame reported the opposite: that NM23 inhibited differentiation. Munoz-Dorado et al 1990 found that *ndk* (*Myxococcus* NM23 homologue, *in vivo*) was essential for growth but was down regulated during development, implying that its presence would inhibit differentiation. Okabe-Kado 1992 showed that *in vitro* a differentiation inhibitory factor (later identified by same group as NM23) inhibited differentiation of mouse leukemia. Yamashiro et al 1994 reported that NM23 levels decrease during differentiation of human megakaryoblasts, consistent with NM23 inhibiting differentiation. Okabe-Kado 1995 showed that recombinant NM23 inhibited erythroid differentiation of leukemia cell lines HEL, KU812, and K562 but not monocyte or granulocyte differentiation of progenitors HL60, U937, or HELlS cells. Venturelli et al 1995 reported that NM23 overexpression inhibited G-CSF dependent granulocyte differentiation of human hematopoietic progenitors. Willems et al 1998 found that NM23 expression decreases as human CD34⁺ hematopoietic progenitors from the bone marrow cells differentiate. In 2002, Willems et al showed that NM23 had no effect on cell proliferation, did not induce or inhibit differentiation but skewed differentiation of CD34+ cells toward the erythroid lineage.

In a 2000 review article, Lombardi summarized these and other contradictory results pertaining to the role of NM23 in differentiation and concluded, "Although the role of the NM23 genes in the control of cell differentiation is widely under investigation, the functional connection between NM23 expression levels and such processes remains to be completely elucidated." In other words, the functional connection between NM23 and differentiation was not understood.

In biological systems, proteins often make up complicated signaling cascades that direct the cell to behave in a particular way. For example, a common way that cells are directed to begin the process of dividing is that a protein (ligand) binds to the extra cellular domain of a transmembrane protein receptor wherein binding of the ligand to the extra cellular domain confers a change in the conformation of the receptor. The ligand-induced conformational change can take place in the extra cellular domain, the intra cellular domain or both and results in a change in which proteins or molecules are able to bind to the receptor. This outside to inside signaling is a common mechanism that is used to signal cells to divide, initiate programmed cell death and many other processes.

One commonly used mechanism that regulates the activity of growth factor receptors is ligand-induced dimerization of the receptor's extra cellular domain which in turn brings the intracellular tails close together which makes a good docking site for modifying proteins such as kinases that initiate a signaling cascade that eventuates in a signal to the cell's nucleus that causes the cell to divide.

Ligand-induced dimerization of the extra cellular domain of growth factor receptors is often accomplished through the binding of ligand dimers; that is two ligands non-covalently bind to each other to form homo- or hetero-dimers which then bind to two receptors that are either the same (homo) or different (hetero).

An important example of ligand-induced receptor dimerization is NM23 dimers binding to and dimerizing the extra cellular domain of MUC1^{∗}, which is the truncated form of the MUC1 transmembrane protein that is tumor and stem cell specific. Whether or not the ligand is a monomer, dimer or a higher order multimer is a function of, among other things, its concentration. For many growth factor receptors, only the dimeric form of the ligand activates the growth factor receptor. Additionally, in primitivebiological systems, there are feedback loops wherein the higher order multimers turn off the function that is promoted by the dimer. For example, the CI protein of Phage lambda turns on transcription of one set of genes when it is bound to DNA as a tetramer but turns off transcription of those genes when, as a function of increased concentration, the CI protein becomes an octamer. Therefore, it would be advantageous to discover if such multimerization-regulated feedback loops that regulate function exist in higher order organisms, such as humans, and to develop methods to manipulate function based on the knowledge of the function of each multimer.

### SUMMARY OF THE INVENTION

In one aspect, the present invention is directed to a method for generating less mature cells from starting cells comprising inducing the starting cells to revert to a less mature state comprising increasing the amount of an NME family member whose multimerization state is the biologically active state or decreasing the relative amount of an NME family member whose multimerization state is the biologically inactive state.

In another aspect, the invention is directed to a method of inhibiting differentiation of embryonic stem cell, induced pluripotent stem cell or progenitor cell comprising increasing the amount of an NME family member whose multimerization state is the biologically active state or decreasing the relative amount of an NME family member whose multimerization state is the biologically inactive state.

In another aspect, the invention is directed to a method of maintaining or inducing pluripotency in cells comprising decreasing the relative amount of an NME family member whose multimerization state is the biologically inactive state.

In yet another aspect, the invention is directed to a method of inhibiting differentiation in embryonic stem cell, induced pluripotent stem cell, or progenitor cell, comprising decreasing the relative amount of an NME family member whose multimerization state is the biologically inactive state.

In any of the methods described above, the NME family member's multimerization state in the biologically inactive state may be a hexamer or higher order multimer. In one aspect, such NME family member may be NME1 (NM23-H1) or NME2 (NM23-H2).

In any of the methods described above, the NME family member's multimerization state in the biologically active state may be a dimer or a monomer that contains two units able to bind to the same target receptor. In this regard, such NME family member may be a mutant or variant of NME1 that favors dimerization or has two binding domains for its cognate ligand, or NME6 or NME7.

In any of the methods described above, the relative amount of the NME family member whose multimerization state is the biologically inactive state may be decreased by adding an NME family member whose multimerization state is the biologically active state. The NME family member whose multimerization state is the biologically active state may be increased by introducing a nucleic acid or small molecule that causes it to be expressed.

And further, in any of the methods described above, the relative amount of the NME family member whose multimerization state is the biologically inactive state may be decreased by introducing nucleic acids or small molecules that down-regulate its expression.

In another aspect, in any of the methods described above, the NME family member whose multimerization state is the biologically inactive state may be decreased and the NME family member whose multimerization state is the biologically active state may be increased by simultaneously introducing a first nucleic acid that down-regulates a first NME that forms the inactive state and a second nucleic acid that up-regulates the NME that forms the active state.

In any of the methods described above, the nucleic acid that down-regulates may be an anti-sense DNA, an inhibitory RNA, siRNA or shRNA and the nucleic acid that up-regulates is an encoding DNA, RNA, mRNA, or plasmid. Such nucleic acid may be modified to facilitate entry into the cell. In this regard, the NME family member that may be down regulated may include NME1, NME2 or NME1 and NME2. In this regard, the NME family member that may be up-regulated may be a mutant or variant of NME1, NME2 that prefers dimerization, NME6 or NME7.

In another aspect, the present invention is directed to a nucleic acid that causes expression of NME1 mutant or variant that prefers dimer formation or has two binding domains of the same ligand; and/or
a. NME7; and/or
b. a nucleic acid that causes expression of NME7; and/or
c. NME6; and/or
d. a nucleic acid that causes expression of NME6; and/or
e. a nucleic acid that down-regulates NME1 or NME1 and NME2.

In still another aspect, the invention is directed to a media for culturing stem or progenitor cells wherein the media contains:
a. NME1 mutant or variant that prefers dimer formation or has two binding domains of the same ligand; and/or
b. a nucleic acid that causes expression of NME1 mutant or variant that prefers dimer formation or has two binding domains of the same ligand; and/or
c. NME7; and/or
d. a nucleic acid that causes expression of NME7; and/or
e. NME6; and/or
f. a nucleic acid that causes expression of NME6; and/or
g. a nucleic acid that down-regulates NME1 or NME1 and NME2.

In yet another aspect, the invention is directed to a media for inducing pluripotency or for inducing cells to revert to a less mature state wherein the media contains:
a. NME1 mutant or variant that prefers dimer formation or has two binding domains of the same ligand; and/or
b. a nucleic acid that causes expression of NME1 mutant or variant that prefers dimer formation or has two binding domains of the same ligand; and/or
c. NME7; and/or
d. a nucleic acid that causes expression of NME7; and/or
e. NME6; and/or
f. a nucleic acid that causes expression of NME6; and/or
g. contains a nucleic acid that down-regulates NME1 or NME1 and NME2.

The media may also contain nucleic acids that encode some or all of the pluripotency-inducing genes or small molecules including OCT4, SOX2, NANOG, KLF4, and c-Myc.

In still another aspect, the invention is directed to a host cell that carries a synthetic nucleic acid that causes decreased expression of an NME family member whose multimerization state is the biologically inactive state. The NME family member may be NME1, NME2 or NME1 and NME2.

In yet another aspect, the invention is directed to a host cell that carries a synthetic nucleic acid that causes increased expression of an NME family member whose multimerization state is the biologically active state. In this regard, the NME family member may include an NME1 mutant or variant that prefers dimer formation or has two binding domains of the same ligand, NME6, or NME7.

In another aspect, the invention is directed to a host cell that carries a synthetic nucleic acid that causes decreased expression of an NME family member whose multimerization state is the biologically inactive state and a synthetic nucleic acid that causes increased expression of an NME family member whose multimerization state is the biologically active state. In this regard, the NME family member whose expression will be decreased may be NME1 or NME1 and NME2 and the NME family member whose expression will be increased may be an NME1 mutant or variant that prefers dimer formation or has two binding domains of the same ligand, and/or NME6, and/or NME7. The cell may also carry a nucleic acid that causes the expression of a gene that is either not expressed in the host cell or is mutated in the host cell. The host cell may also carry a nucleic acid to down-regulate an unwanted gene and up-regulates a desired or corrected gene. The host cell may be an embryonic stem cell, induced pluripotent stem cell, progenitor cell, or somatic cell.

In another aspect, the invention is directed to a method of treating a patient wherein the cells described above are administered to the patient. The cells may be administered by bone marrow transplant, transplant into a specific site, transfusion, injection, or topical treatment. The treatment may be for any disease or condition that would be alleviated by treatment with stem cells, progenitors, or by correction of a genetic abnormality or defect. Or, the cells may be differentiated prior to administration to patient and wherein NME1, NME6 or NME7 are withdrawn during differentiation process.

In yet another aspect, the invention is directed to a method of treating cancer in a patient comprising administering an agent to the patient such that the agent increases the amount of an NME family member whose multimerization state is the biologically inactive state or decreases the relative amount of an NME family member whose multimerization state is the biologically active state. In this regard, the NME family member whose multimerization state is the biologically active state may be NME7, and the NME family member whose multimerization state is the biologically inactive state may be NME1, NME2 or NME8.

### BRIEF DESCRIPTION OF THE DRAWINGS

The present invention will become more fully understood from the detailed description given herein below, and the accompanying drawings which are given by way of illustration only, and thus are not limitative of the present invention, and wherein;

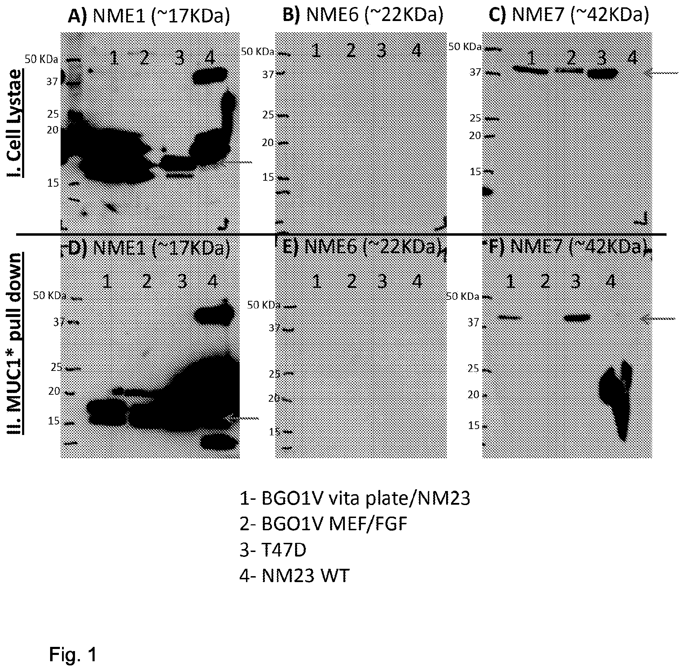

Figures 1A-F show photographs of Western blots detecting the presence of NME1 (A,D), NME6 (B,E) or NME7 (C,F) in human stem cells cultured in NM23-S120G dimers, cultured in bFGF over MEFs or human breast cancer cells or the presence of the NME isoforms in a MUC1^{∗} pull-down assay.

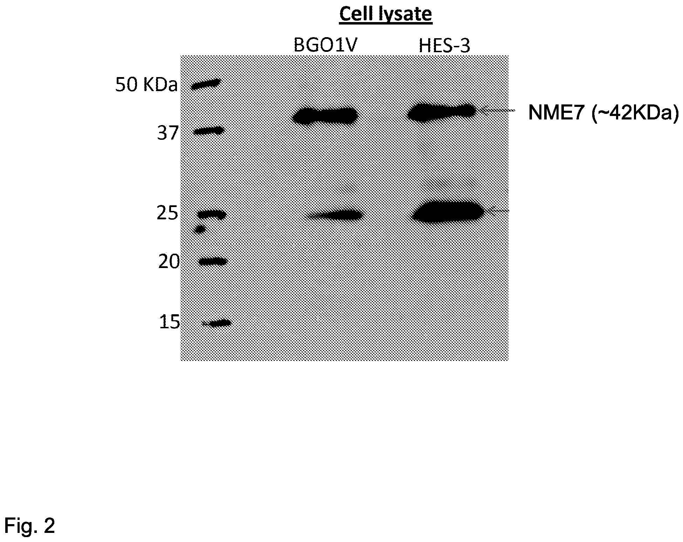

Figure 2 is a photograph of a Western blot of human embryonic stem cell lysates probed with an antibody specific for NME7.

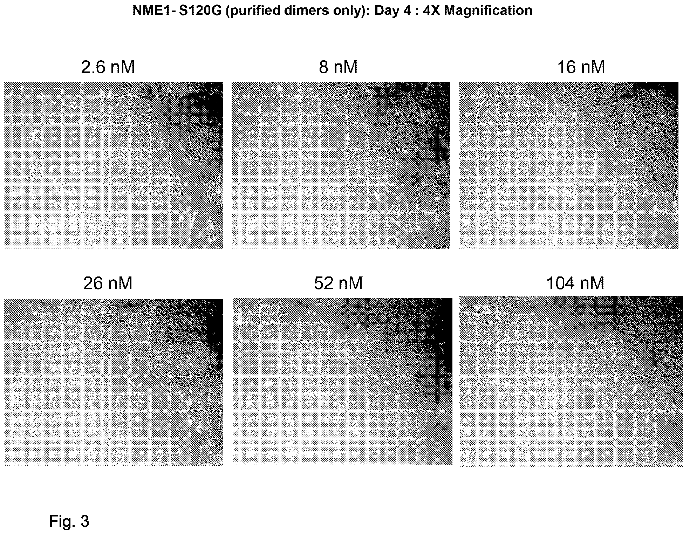

Figure 3 shows photographs of human BGO1v embryonic stem cells that remain pluripotent and undifferentiated after several days in culture in NME1-S120G dimers present at a range of concentrations.

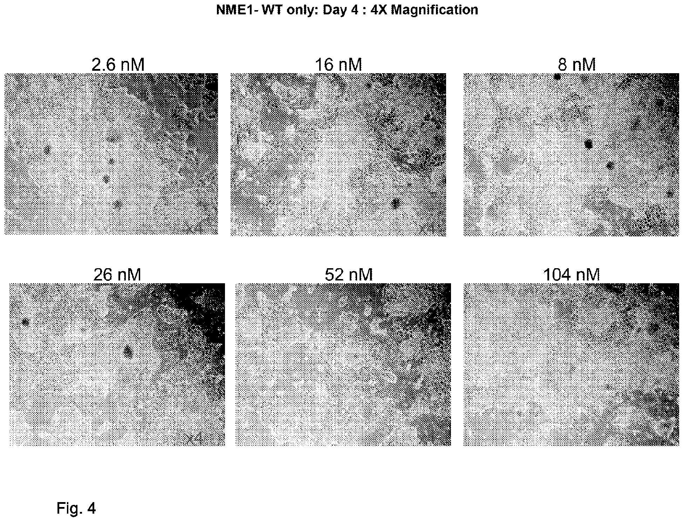

Figure 4 shows photographs of human BGO1v embryonic stem cells that have differentiated and are no longer pluripotent after several days in culture in NME1-wild type hexamers present at a range of concentrations.

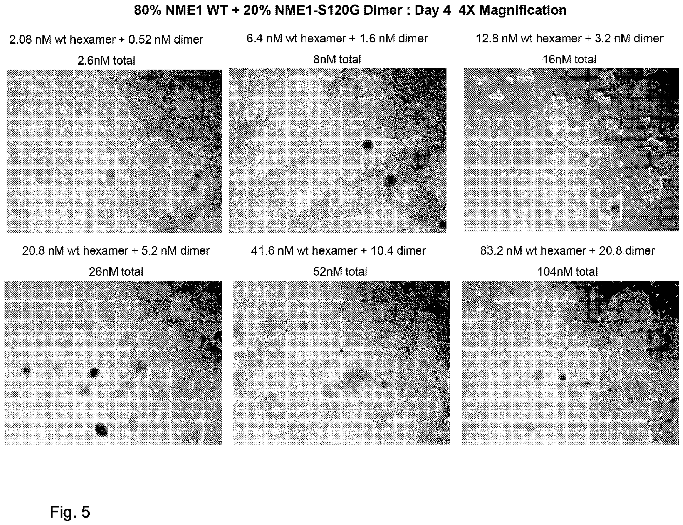

Figure 5 shows photographs of human BGO1v embryonic stem cells that have differentiated and are no longer pluripotent after several days in culture in a mixture of 20% NME1-S120G dimers and 80% NME1-wild type hexamers present at a range of concentrations.

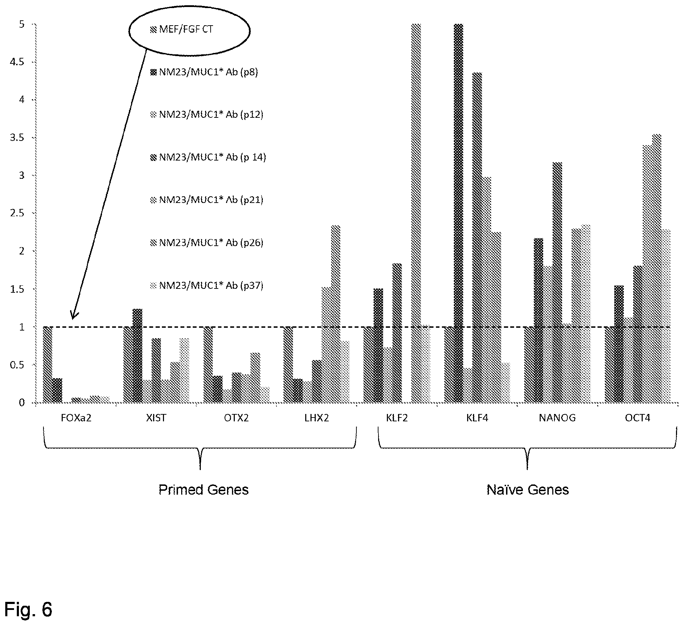

Figure 6 is a graph showing quantitative PCR measurements of the expression levels of pluripotent, naive and primed genes in human embryonic H9 stem cells cultured for up to 37 passages in NME1-S120G dimers, compared to the same source cells cultured in bFGF.

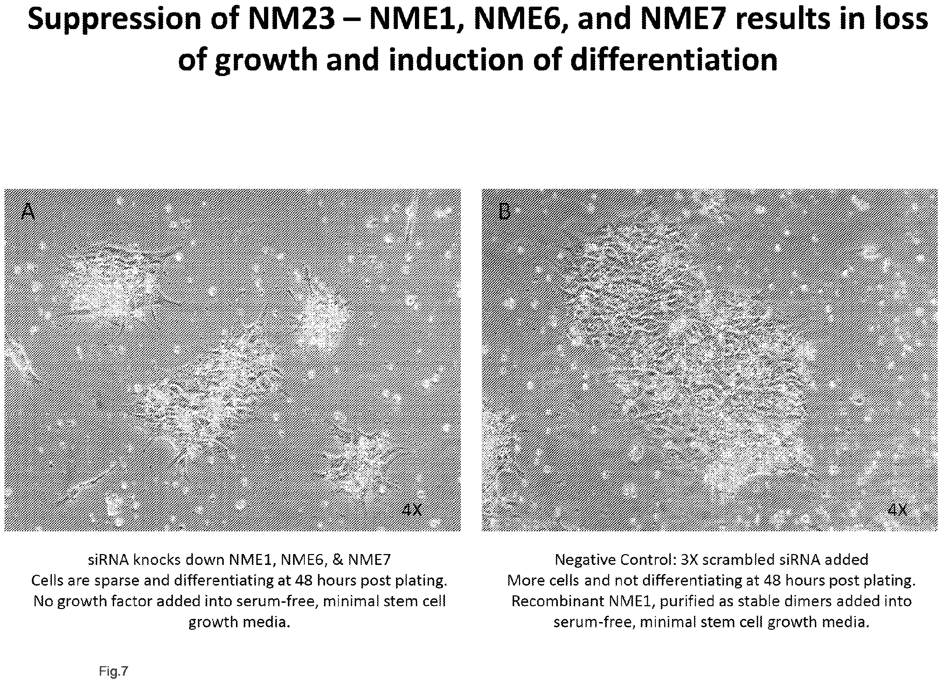

Figures 7A-B show photographs of human stem cells in which siRNAs specific for NME1, NME6 and NME7 have been used to suppress expression of the three NM23 isoforms.

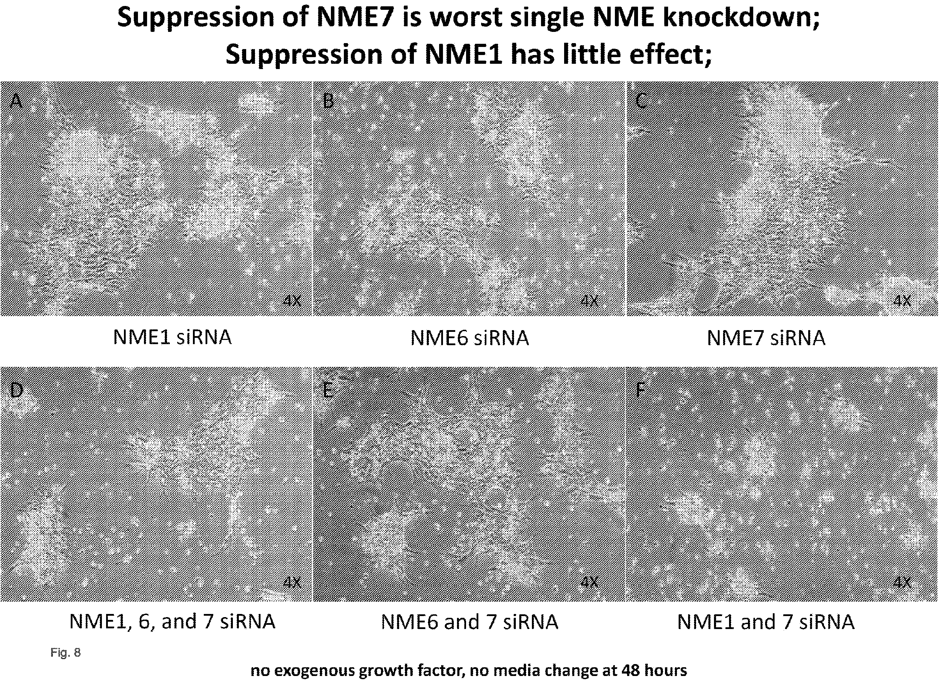

Figures 8A-F show photographs of human stem cells in which siRNAs specific for NME1, NME6 and NME7 have been used to suppress expression of the three NM23 isoforms either separately or in combinations.

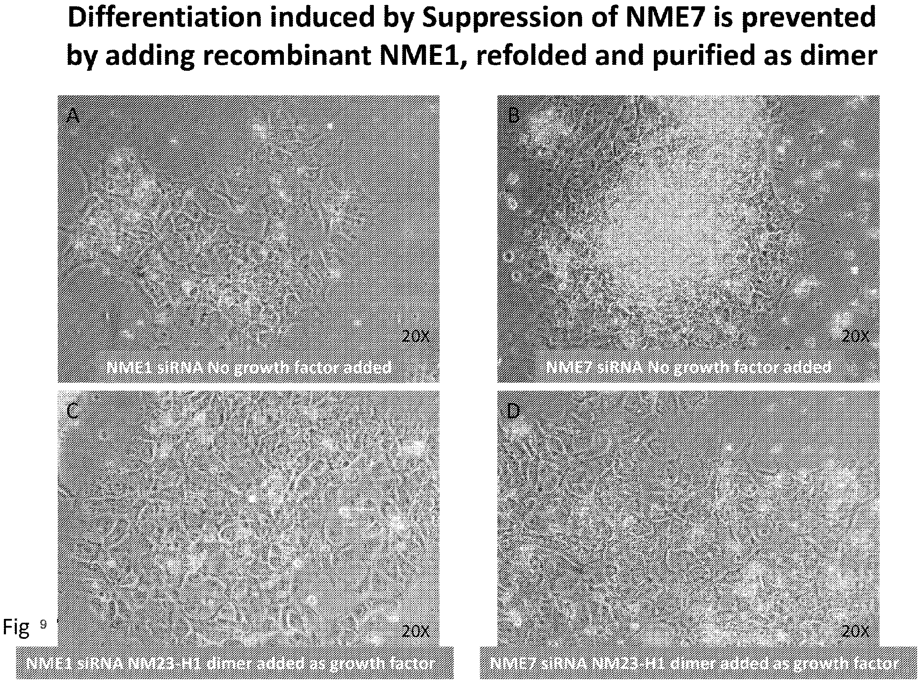

Figures 9A-D show photographs of human stem cells in which differentiation induced by suppression of NME7 is rescued if cells are treated with recombinant NME1 purified as stable dimers.

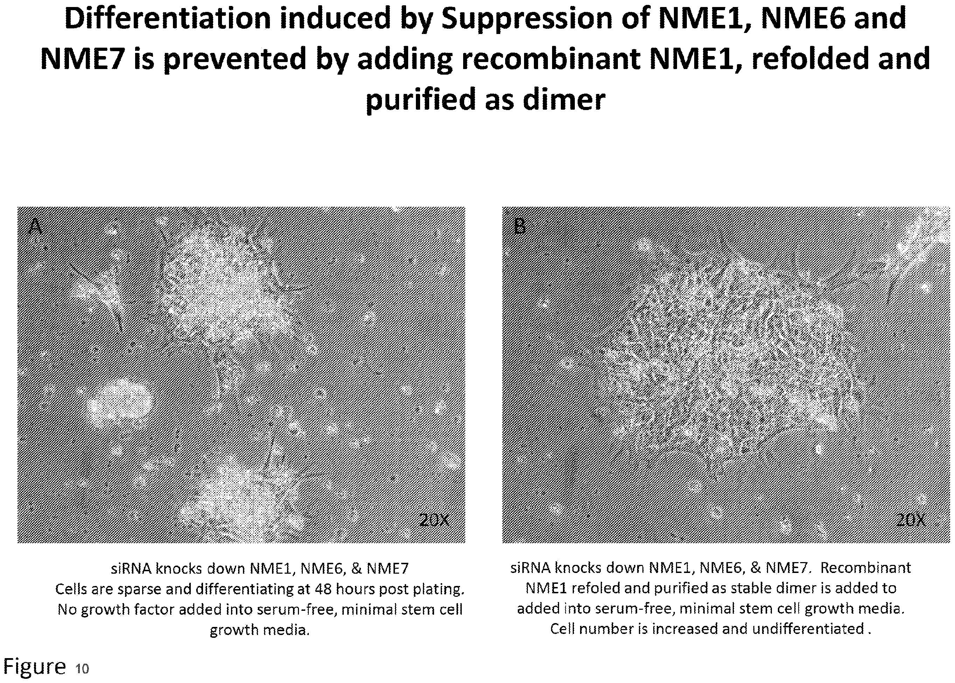

Figure 10A-B show photographs of human stem cells in which siRNAs specific for NME1, NME6 and NME7 have been used to suppress expression of all three NM23 isoforms and that inhibition of cell growth and differentiation induced by suppressing all three NMEs is rescued by treating cells with recombinant NME1 purified as stable dimers.

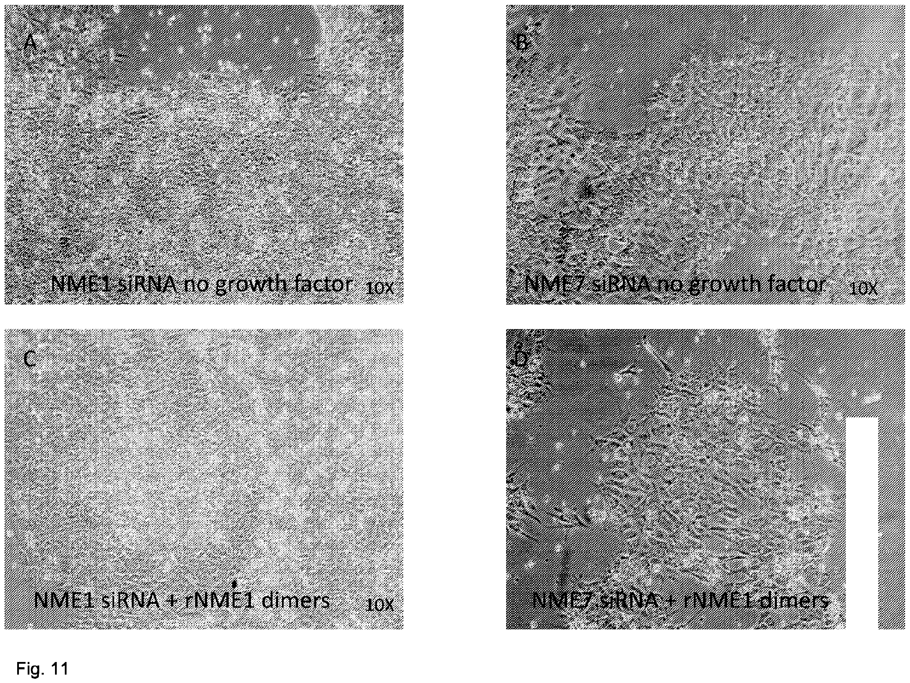

Figures 11A-D show magnified photographs of human stem cells in which expression of NME1 (A,C) or NME7 (B,D) has been suppressed using siRNAs, or suppressed but wherein the suppressed cells are treated with recombinant NME1 purified as stable dimers (C,D).

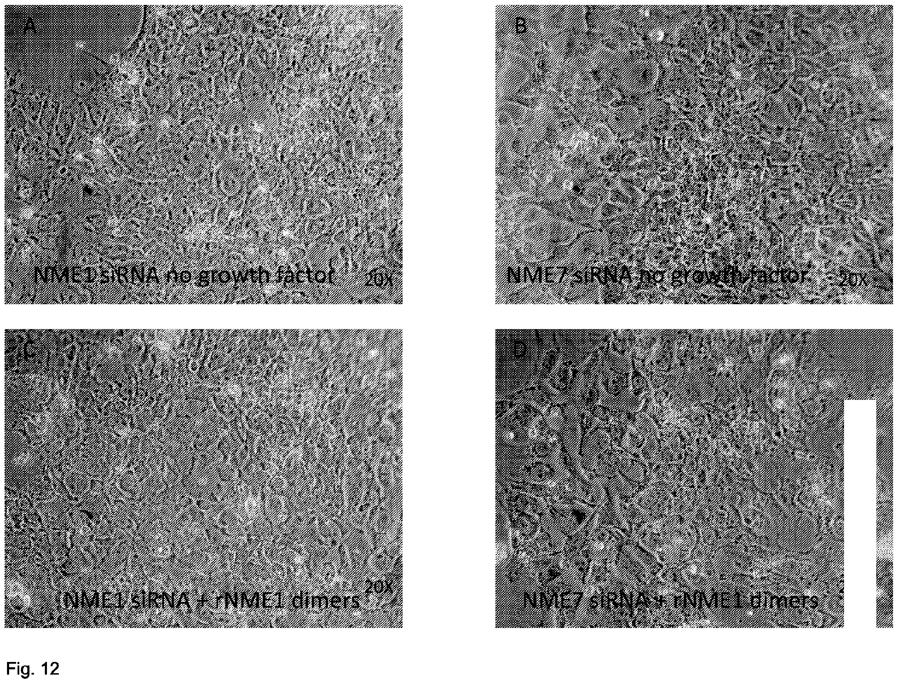

Figures 12A-D show magnified photographs of human stem cells in which expression of NME1 (A,C) or NME7 (B,D) has been suppressed using siRNAs, or suppressed but rescued by also treating cells with recombinant NME1 purified as stable dimers (C,D).

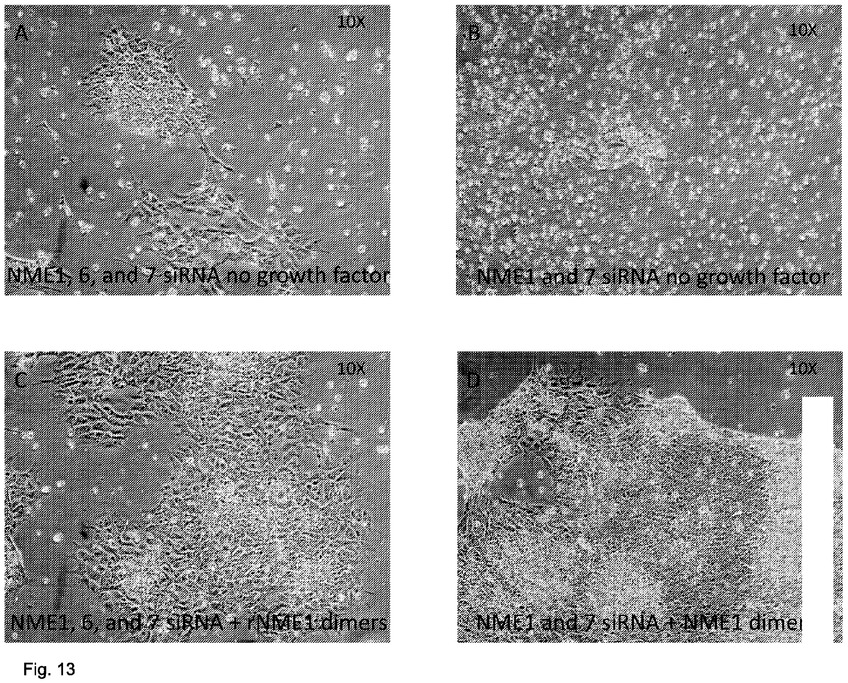

Figures 13A-D show magnified photographs of human stem cells in which expression of NME1, NME6 and NME7 are suppressed (A,C) or NME1 and NME7 are suppressed (B,D), wherein cells are rescued to some extent by culturing the cells in recombinant NME1 purified as stable dimers (C,D).

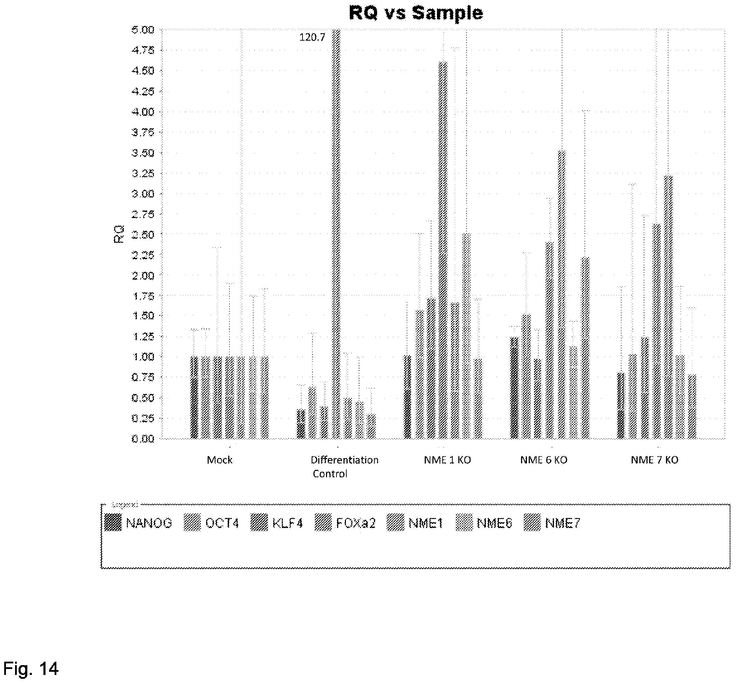

Figure 14 shows a graph of an RT-PCR experiment in which expression of pluripotency genes NANOG, OCT4 and KLF4 or differentiation marker FOXA2, plus NME1, NME6 and NME7 genes are measured in human stem cells in which either NME1, NME6 or NME7 has been suppressed or "Knocked Out"; controls are a mock transfection and the same stem cells that have differentiated. A striking increase in differentiation marker FOXA2 is seen in NME knock out cells and in the differentiation control. A significant increase in the expression of NME1 is observed when NME6 or NME7 is suppressed.

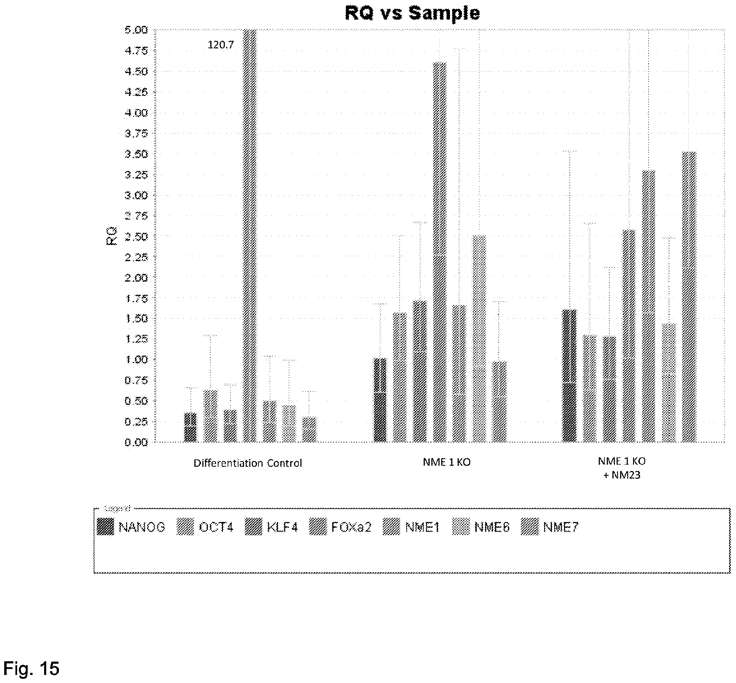

Figure 15 shows a graph of an RT-PCR experiment in which expression of pluripotency genes NANOG, OCT4 and KLF4 or differentiation marker FOXA2, plus NME1, NME6 and NME7 genes are measured in human stem cells in which NME1 has been Knocked Out (KO) wherein one set of NME1 suppressed cells has been cultured in media supplemented with human recombinant NME1 dimer form; control is the same stem cells that have been allowed to differentiate. A striking increase in differentiation marker FOXA2 is seen in NME1 knock out cells and in the differentiation control.

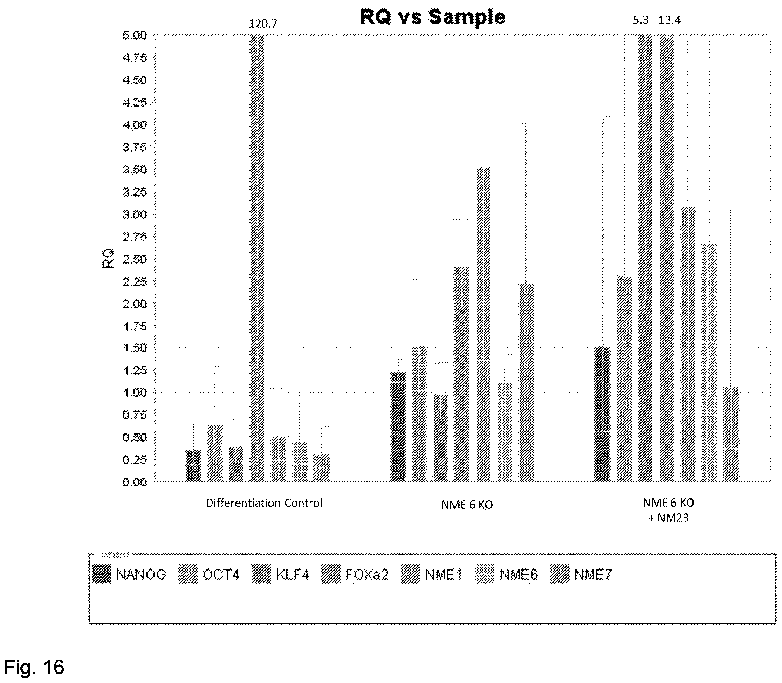

Figure 16 shows a graph of an RT-PCR experiment in which expression of pluripotency genes NANOG, OCT4 and KLF4 or differentiation marker FOXA2, plus NME1, NME6 and NME7 genes are measured in human stem cells in which NME6 has been Knocked Out (KO) wherein one set of NME6 suppressed cells has been cultured in media supplemented with human recombinant NME1 dimer form; control is the same stem cells that have been allowed to differentiate.

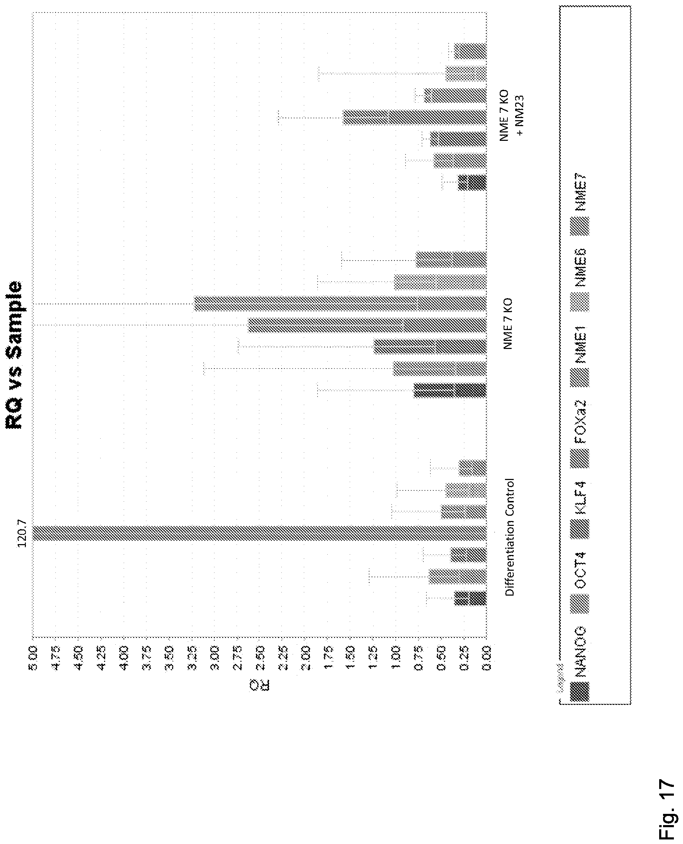

Figure 17 shows a graph of an RT-PCR experiment in which expression of pluripotency genes NANOG, OCT4 and KLF4 or differentiation marker FOXA2, plus NME1, NME6 and NME7 genes are measured in human stem cells in which NME7 has been Knocked Out (KO) wherein one set of NME7 suppressed cells has been cultured in media supplemented with human recombinant NME1 dimer form; control is the same stem cells that have been allowed to differentiate. A striking increase in differentiation marker FOXA2 is seen in NME7 suppressed cells in addition to a significant increase in the expression of NME1. Both return to an almost normal state when the suppressed cells are cultured in a minimal media supplemented with NME1 in dimer form.

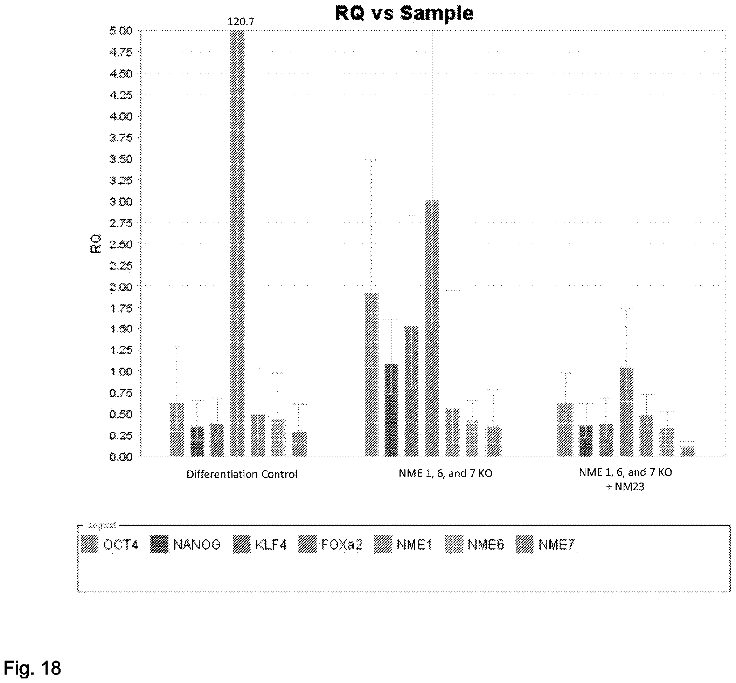

Figure 18 shows a graph of an RT-PCR experiment in which expression of pluripotency genes NANOG, OCT4 and KLF4 or differentiation marker FOXA2, plus NME1, NME6 and NME7 genes are measured in human stem cells in which NME1, NME6 and NME7 have been suppressed (KO) wherein one set of suppressed cells has been cultured in media supplemented with human recombinant NME1 dimer form; control is the same stem cells that have been allowed to differentiate. A striking increase in differentiation marker FOXA2 is seen in the suppressed cells, wherein suppressed cells that are cultured in a minimal media supplemented with NME1 in dimer form return to a near normal gene profile.

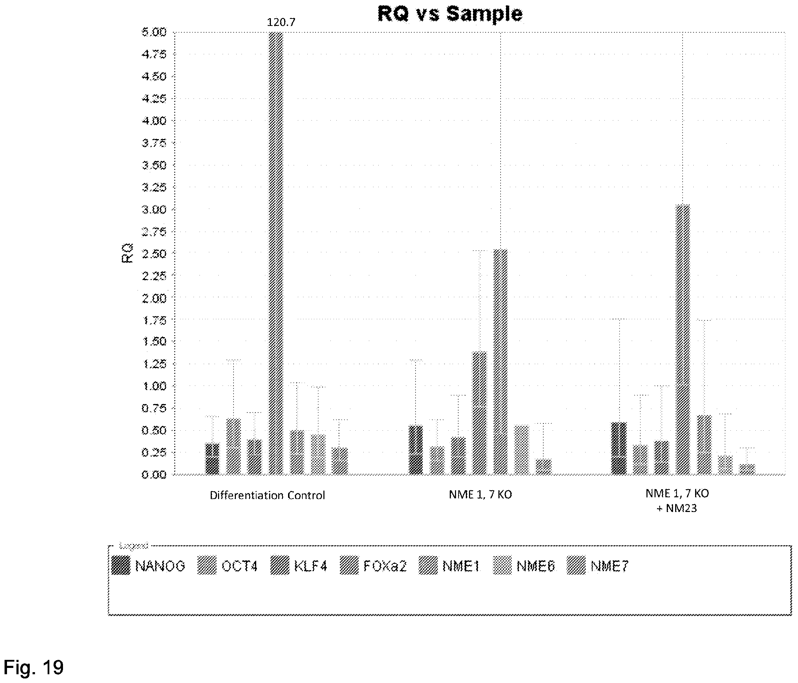

Figure 19 shows a graph of an RT-PCR experiment in which expression of pluripotency genes NANOG, OCT4 and KLF4 or differentiation marker FOXA2, plus NME1, NME6 and NME7 genes are measured in human stem cells in which NME1 and NME7 have been suppressed (KO) wherein one set of suppressed cells has been cultured in media supplemented with human recombinant NME1 dimer form; control is the same stem cells that have been allowed to differentiate.

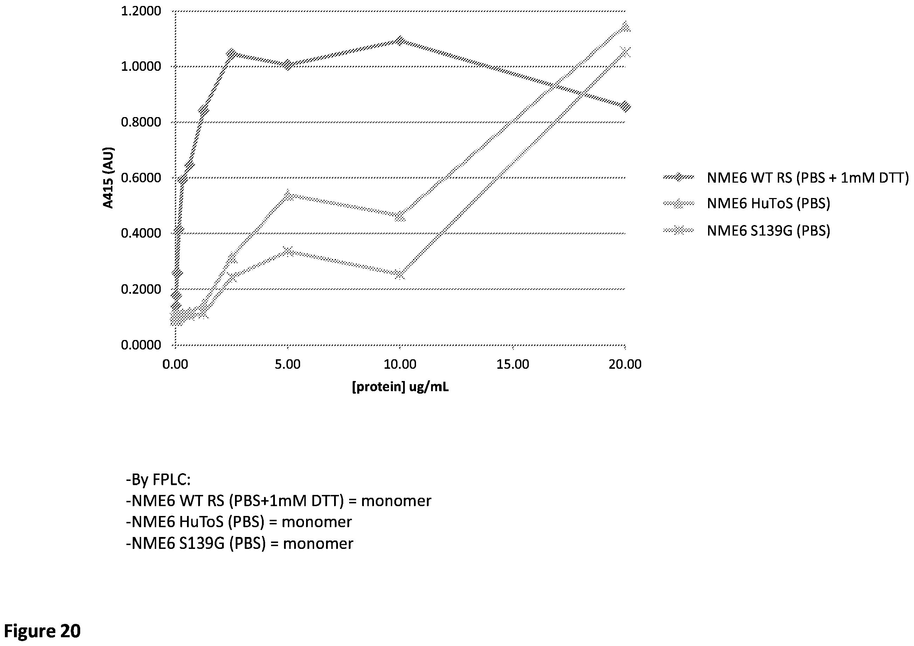

Figure 20 shows a graph of an ELISA assay in which a synthetic PSMGFR peptide of the MUC1^{∗} extra cellular domain is immobilized on the surface of a multi-well plate and three different recombinant human NME6 proteins are assayed for binding to the peptide, wherein one is the wild type human NME6 that has been denatured and refolded (NME6 WT RS), the second is a human NME6 wherein the region that contributes to dimerization is swapped to a sea sponge sequence (NME6 HuToS), and the third is a mutant NME6 S139G, which is in a comparable position to the NME1 mutant identified in cancers that prefers dimerization. All demonstrate binding to the peptide of the extra cellular domain of MUC1^{∗}.

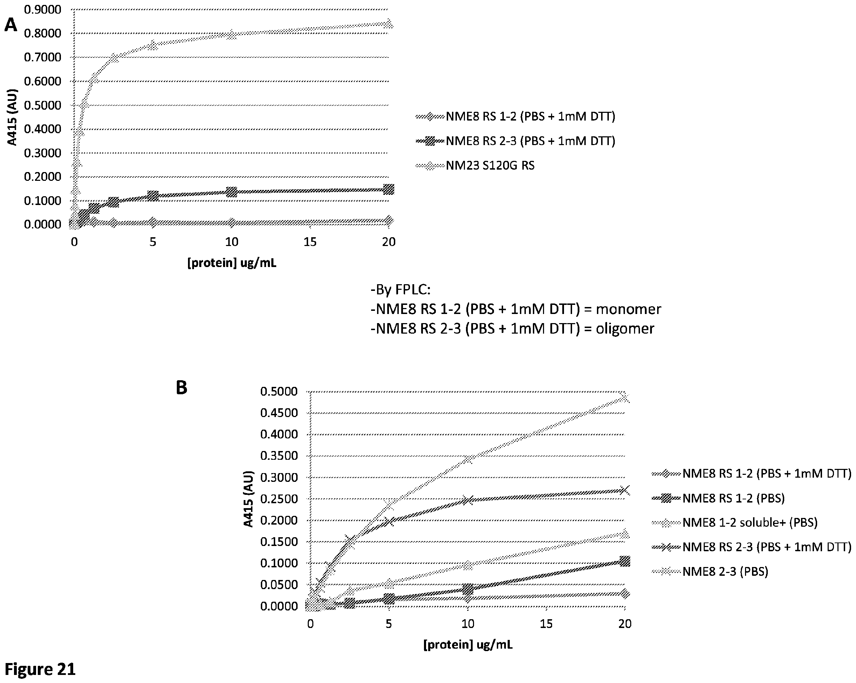

Figures 21A-B show graphs of ELISA assays in which a synthetic PSMGFR peptide of the MUC1^{∗} extra cellular domain is immobilized on the surface of a multi-well plate and different recombinant human NME8 proteins are assayed for binding to the peptide, wherein one is a construct containing only NME8 domains A and B (NME8 1-2) and the other is a construct containing only NME8 domains B and C (NME8 2-3). The NME variants tested were either expressed in soluble form or were refolded (RS). In some cases, DTT was added to break up large oligomers. All demonstrate binding to the peptide of the extra cellular domain of MUC1^{∗}. NM23 S120G dimers are added for comparison to the binding of the NME8 constructs (A).

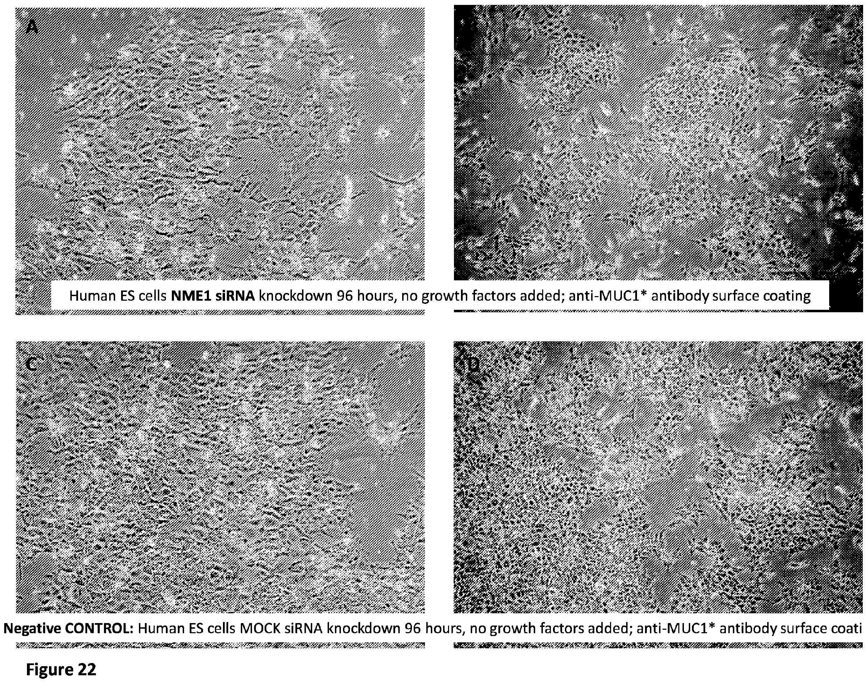

Figures 22A-D show magnified photographs of human stem cells at 96 hours post transfection of siRNA to suppress NME1 expression (A,B) and control cells wherein transfection reagents were added but with either no siRNA or a scrambled sequence siRNA (C,D). Comparison of NME1 suppressed cells to the control cells shows very little difference. Note that stem cells were plated over a layer of bivalent anti-MUC1^{∗} antibody which also functions as a MUC1^{∗} stimulating growth factor.

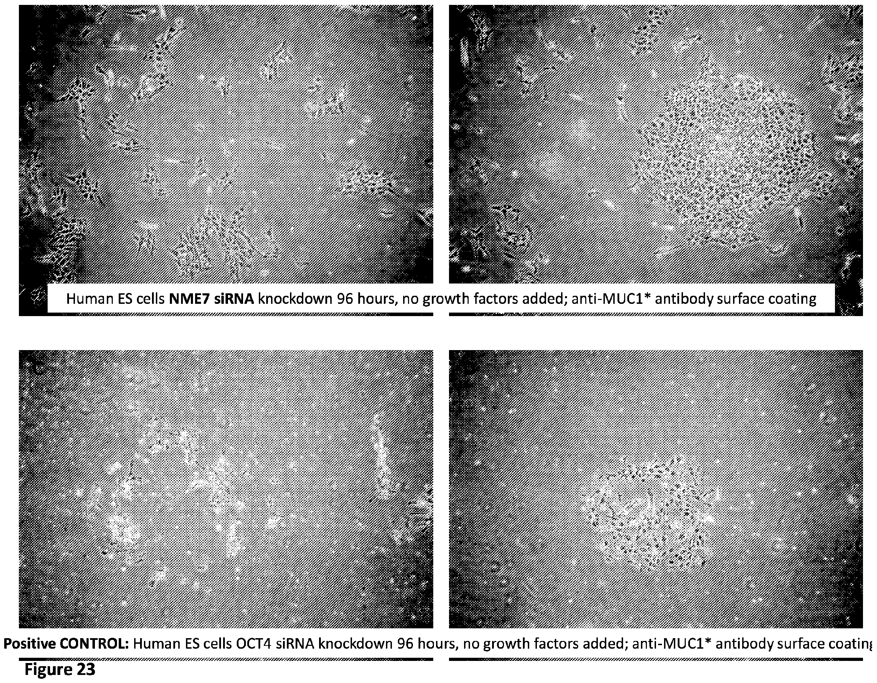

Figures 23A-D show magnified photographs of human stem cells at 96 hours post transfection of siRNA to suppress NME7 expression (A,B) and control cells wherein pluripotency gene Oct4 was suppressed (C,D). Comparison of NME7 suppressed cells to the OCT4 suppressed cells shows very little difference, wherein both have severely inhibited cell viability and remaining cells have taken on a fibroblast morphology.

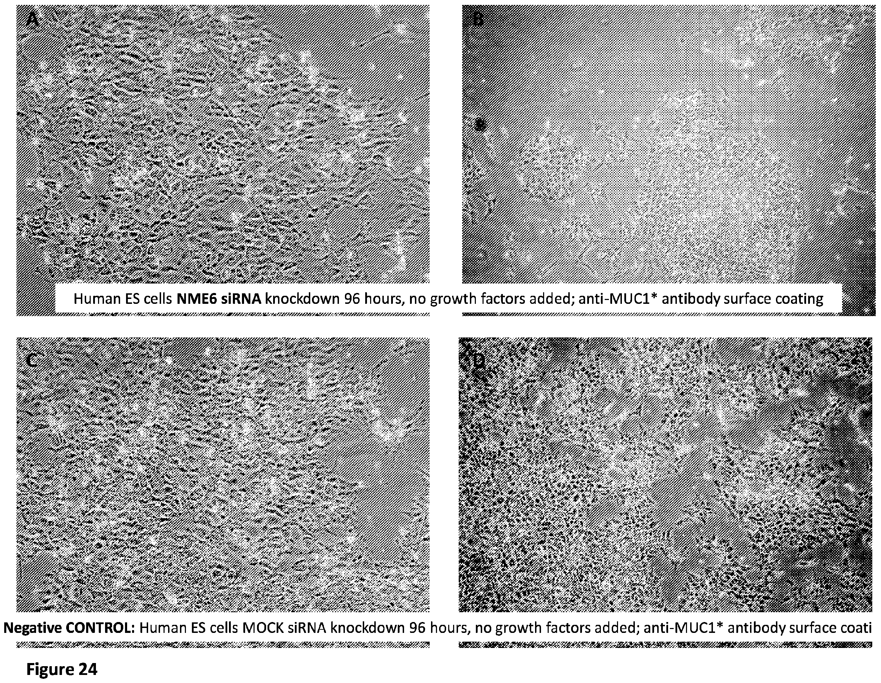

Figure 24 shows magnified photographs of human stem cells at 96 hours post transfection of siRNA to suppress NME6 expression (A,B) and control cells wherein transfection reagents were added but with either no siRNA or a scrambled sequence siRNA (C,D). Comparison of NME6 suppressed cells to the control cells shows very little difference, although NME6 suppressed cells had areas of differentiating cells as can be seen as the brighter, thickening center of the cell cluster in Panel B. Note that stem cells were plated over a layer of bivalent anti-MUC1^{∗} antibody which also functions as a MUC1^{∗} stimulating growth factor.

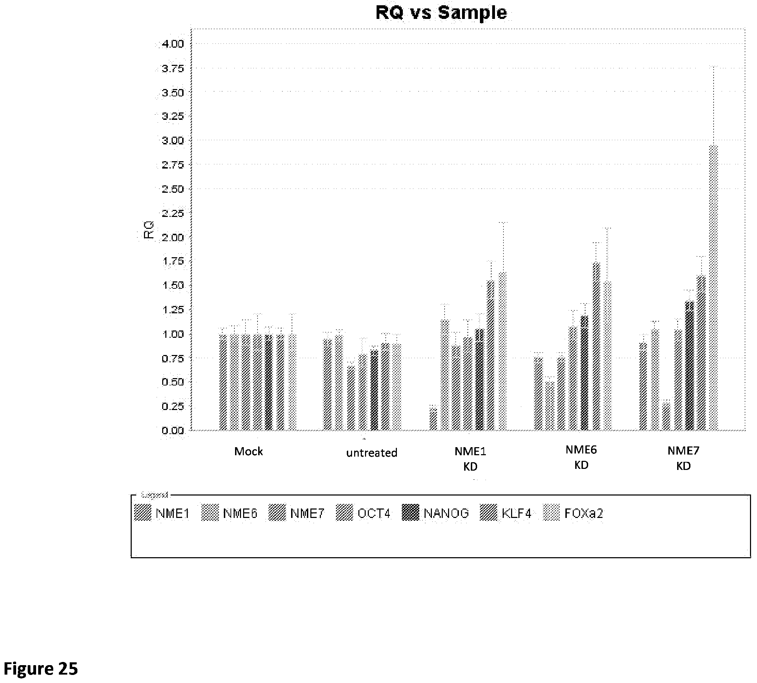

Figure 25 shows a graph of an RT-PCR experiment in which expression of pluripotency genes NANOG, OCT4 and KLF4 or differentiation marker FOXA2, plus NME1, NME6 and NME7 genes are measured in human stem cells 96 hours post transfection of siRNA to suppress NME1, NME6 or NME7. A minimal media free of growth factors, serum or cytokines was changed every 48 hours, but note that stem cells were plated over a layer of bivalent anti-MUC1^{∗} antibody which also functions as a MUC1^{∗} stimulating growth factor. Suppression of NME7 shows a marked increase in the expression of differentiation marker FOXA2.

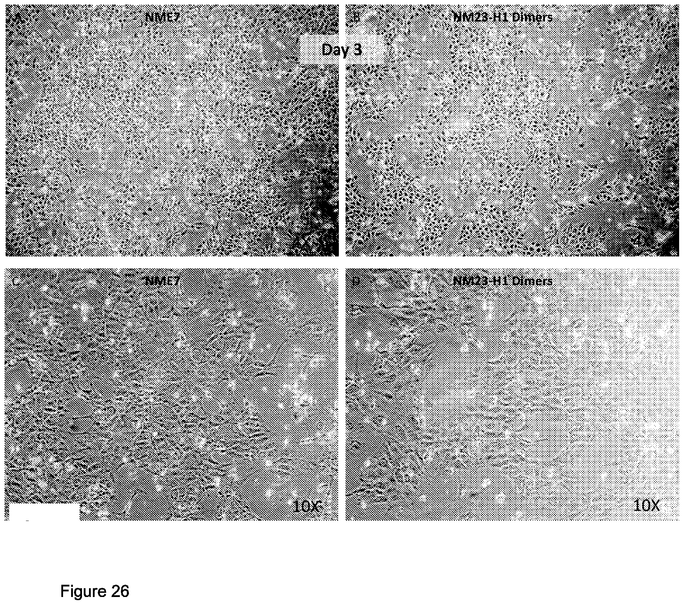

Figures 26A-D show magnified photographs of human HES-3 stem cells that have been cultured in a minimal media free of growth factors, serum or cytokines and supplemented with either NME7 monomers (A,C) or NME1 (NM23-H1) dimers (B,D).

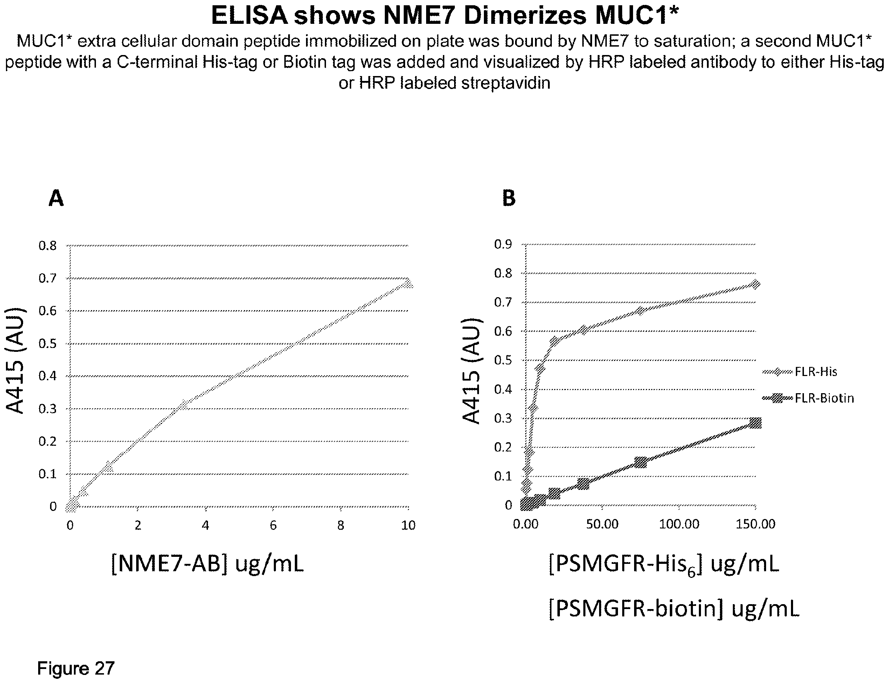

Figures 27A-B show graphs of an ELISA sandwich assay that shows that NME7 monomers have two binding sites for the PSMGFR peptide of the MUC1^{∗} extra cellular domain. A synthetic PSMGFR peptide is immobilized on the surface of a multi-well plate and recombinant human NME7 (A and B domains and devoid of the M leader sequence) proteins are assayed for binding to the peptide (A), then bound by a histidine-tagged PSMGFR peptide, which is detected using an antibody to its histidine tag (B).

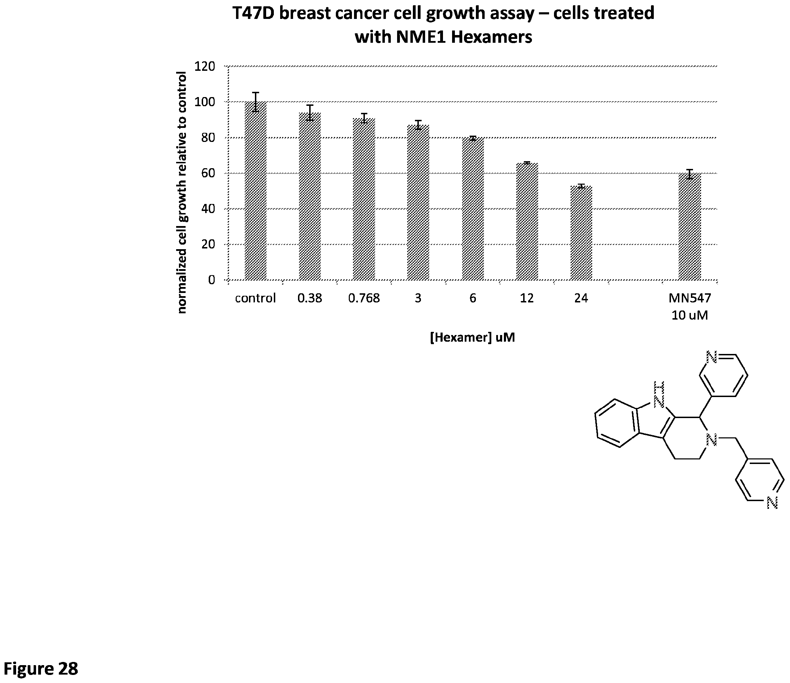

Figure 28 shows a graph of T47D breast cancer cell growth as a function of concentration of NME1 hexamers; experiment shows NME1 hexamers inhibit cancer cell growth.

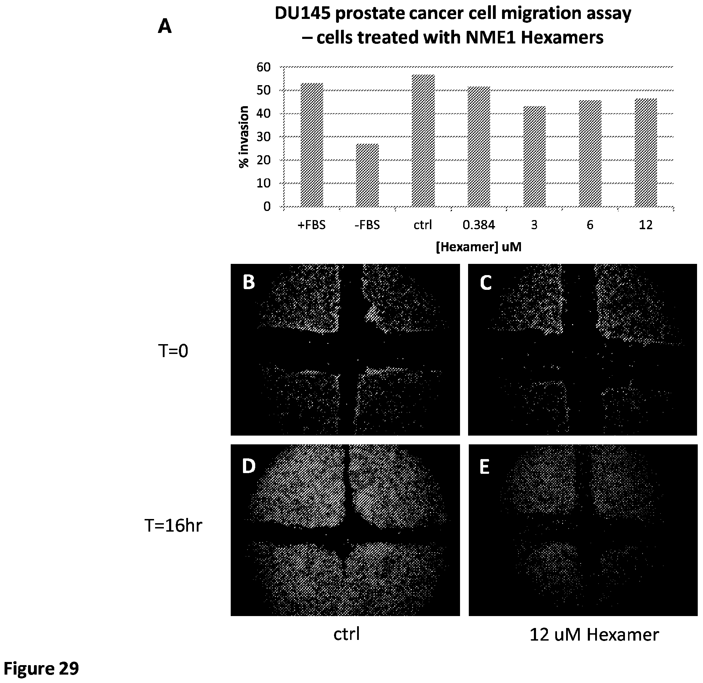

Figures 29A-E show a graph of percent invasion of DU145 prostate cancer cells in response to treatment with NME1 hexamers, over a range of concentrations (A) and photographs of the cell migration assay wells (B-E) in which a cross is etched across a field of growing cancer cells and their ability to migrate across the gap is measured as a function of time. Photographs were taken at time zero (B,C) and at 16 hours (D,E), wherein NME1 hexamers at 12 uM were added to the cells (C,E) and buffer alone was added to control cells (B,D). NME1 hexamers inhibited cancer cell migration.

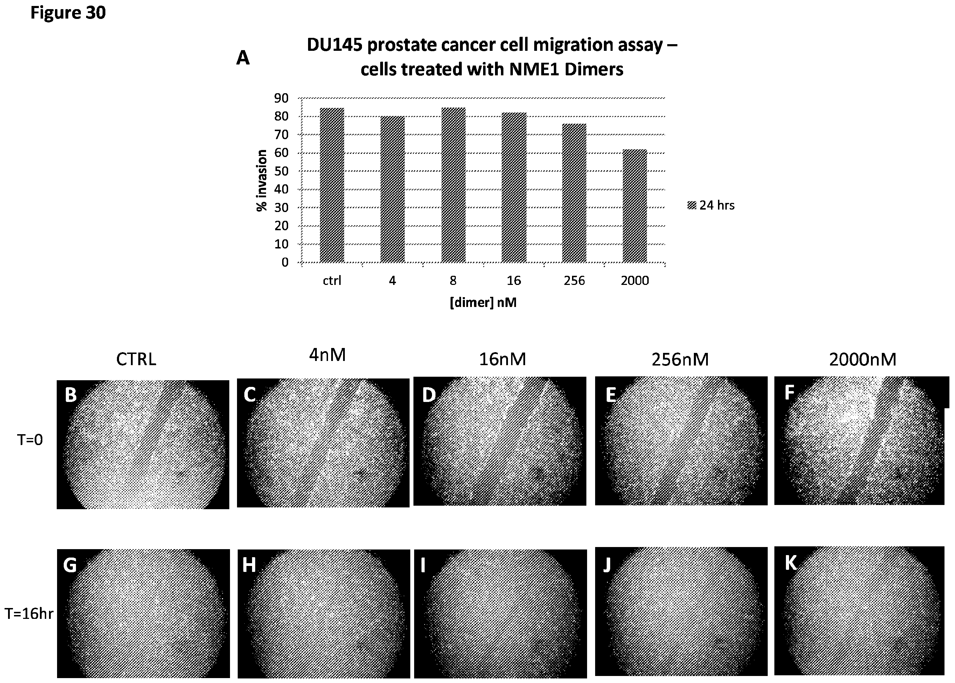

Figures 30A-K show a graph of percent invasion of DU145 prostate cancer cells in response to treatment with NME1 dimers, over a range of concentrations (A) and photographs of the cell migration assay wells (B-K) in which a slash mark is etched across a field of growing cancer cells at time zero (B-F) and their ability to migrate across the gap is measured at 16 hours (G-K). NME1 dimers did not inhibit cancer cell migration. Note that NME1 dimers induce cancer cell growth at optimal concentrations (4-16 nM) wherein one nME1 dimer dimerizes two MUC1^{∗} receptors; at very high concentrations, each NME1 dimer binds to a single MUC1^{∗} receptor and inhibits cancer cell growth and migration.

### DETAILED DESCRIPTION OF THE PREFERRED EMBODIMENTS

In the present application, "a" and "an" are used to refer to both single and a plurality of objects.

As used herein, "biologically active" NME multimer includes NME multimer that induces the starting cells to revert to a less mature state or inhibits differentiation of immature cells, or maintains the immature state of a cell.

As used herein, "biologically inactive" NME multimer includes NME multimer that does not induce the starting cells to revert to a less mature state or does not inhibit differentiation of immature cells, or does not maintain an immature state of the cell.

As used herein, "increasing MUC1^{∗} activity" refers to directly or indirectly increasing MUC1^{∗} signaling, and includes without limitation the dimerization of MUC1^{∗} receptor and also increased production of MUC1^{∗} by cleavage of the MUC1 receptor. MUC1^{∗} activity may be also increased by higher transcriptional expression of MUC1 receptor, which is further cleaved and dimerized. Therefore, in one aspect, MUC1^{∗} activity may be increased by a higher activity of the effector molecule that dimerizes MUC1*, or the higher activity of the cleavage molecule that cleaves MUC1 so that MUC1^{∗} is formed, or increased expression of the MUC1. Therefore, any chemical or biological species that is able to increase the activity of the MUC1^{∗} dimerizing ligand, MUC1 cleavage enzyme to form MUC1^{∗}, or any transcriptional activator that enhances expression of MUC1, is encompassed as a species that "increases MUC1^{∗} activity".

As used herein, "MUC1 Growth Factor Receptor" (MGFR) is a functional definition meaning that portion of the MUC1 receptor that interacts with an activating ligand, such as a growth factor or a modifying enzyme such as a cleavage enzyme. The MGFR region of MUC1 is that extracellular portion that is closest to the cell surface and is defined by most or all of the PSMGFR, as defined below. The MGFR is inclusive of both unmodified peptides and peptides that have undergone enzyme modifications, such as, for example, phosphorylation, glycosylation and so forth.

As used herein, "Primary Sequence of the MUC1 Growth Factor Receptor" (PSMGFR) refers to peptide sequence that defines most or all of the MGFR in some cases, and functional variants and fragments of the peptide sequence. The PSMGFR is defined as SEQ ID NO:6, and all functional variants and fragments thereof having any integer value of amino acid substitutions up to 20 (i.e. 1, 2, 3, 4, 5, 6, 7, 8, 9, 10, 11, 12, 13, 14, 15, 16, 17, 18, 19, or 20) and/or any integer value of amino acid additions or deletions up to 20 at its N-terminus and/or C-terminus. A "functional variant or fragment" in the above context refers to such variant or fragment having the ability to specifically bind to, or otherways specifically interact with, ligands that specifically bind to, or otherwise specifically interact with, the peptide of SEQ ID NO:6, while not binding strongly to identical regions of other peptide molecules identical to themselves, such that the peptide molecules would have the ability to aggregate (i.e. self-aggregate) with other identical peptide molecules. One example of a PSMGFR that is a functional variant of the PSMGFR peptide of SEQ NO:6 is SEQ ID NO:8, which differs from SEQ ID NO:6 by including an -SPY- sequence instead of the -SRY-.

As used herein, "MUC1*" refers to the MUC1 protein with the N-terminus truncated such that the extracellular domain is essentially comprised of the PSMGFR (SEQ ID NO:5).

As used herein "MUC1^{∗} associated factors" refers to agents that modify, activate, modulate the activity of, or modulate the expression of MUC1^{∗}. MUC1^{∗} associated factors include, without limitation, agents that affect dimerization of MUC1^{∗} receptor, increased production of MUC1^{∗}, induce cleavage of the MUC1 receptor, agents that increase MUC1^{∗} activity by higher transcriptional expression of MUC1 receptor, which is further cleaved and dimerized.

As used herein, "effective amount" is an amount sufficient to effect beneficial or desired clinical or biochemical results. An effective amount can be administered one or more times. For purposes of this invention, an effective amount of an inhibitor compound is an amount that is sufficient to induce or maintain pluripotency of a cell or activate MUC1^{∗}

As used herein, "fragments" or "functional derivatives" refers to biologically active amino acid sequence variants and fragments of the native ligands or receptors of the present invention, as well as covalent modifications, including derivatives obtained by reaction with organic derivatizing agents, post-translational modifications, derivatives with nonproteinaceous polymers, and immunoadhesins.

As used herein, "immature" cells refers to cells that can undergo at least one more step of differentiation and expresses markers of a particular cell type that is known to be able to undergo at least one more step of differentiation.

As used herein, "cell having less mature state than starting cell" refers to a cell that has de-differentiated so that it has an increased ability to differentiate into a different cell type than the starting cell or has an increased ability to differentiate into more cell types than the starting cell. A cell in a less mature state can be identified by measuring an increase in the expression of pluripotency markers, by a determination that the expression levels of pluripotency markers are closer to those of pluripotent stem cells or by measuring markers of a less mature state than the starting cells. For example, hematopoietic stem cells that can differentiate into any blood cell type are characterized by the expression of CD34 and the absence of CD38. As these cells differentiate, they go from CD34+/CD38- to CD34+/CD38-then to CD34-/CD38+. If one were to induce the CD34-/CD38+ cells to revert to a less mature state, the cells would regain expression of CD34. The technique of transdifferentiation involves reverting starting cells to a less mature state wherein the cells become unstable and can be directed to differentiate into a differentiate cell type than the starting cell, even if the starting cell was at the same relative level of differentiation as the resultant cell (Ieda et al 2010; Efe et al 2011). For example, cardio fibroblasts have been reverted to a less mature state by brief ectopic expression of OCT4, SOX2, KLF4 and c-MYC, then from this unstable state, differentiated into cardiomyocytes.

As used herein, "ligand" refers to any molecule or agent, or compound that specifically binds covalently or transiently to a molecule such as a polypeptide. When used in certain context, ligand may include antibody. In other context, "ligand" may refer to a molecule sought to be bound by another molecule with high affinity, such as but not limited to a natural or unnatural ligand for MUC1^{∗} or a cleaving enzyme binding to MUC1 or MUC1^{∗} or a dimerizing ligand for MUC1^{∗}.

As used herein, "Naive stem cells" are those that resemble and share quantifiable characteristics with cells of the inner mass of a blastocyst. Naive stem cells have quantifiable differences in expression of certain genes compared to primed stem cells, which resemble and share traits and characteristics of cells from the epiblast portion of a blastocyst. Notably, naive stem cells of a female source have two active X chromosomes, referred to as XaXa, whereas the later primed stem cells of a female source have one of the X chromosomes inactivated (Nichols and Smith, ).

As used herein, "NME" family proteins is a family of ten (10) proteins, some of which have been recently discovered, wherein they are categorized by their shared sequence homology to nucleoside diphosphate kinase (NDPK) domains, even though many of the NME family members are incapable of kinase activity. NME proteins were previously known as NM23-H1 and NM23-H2 then NM23-H3 through NM23-10 as they were being discovered. The different NME proteins function differently. Herein, NME1 and NME6 bind to and dimerize the MUC1^{∗} receptor (wherein its extra cellular domain is comprised essentially of the PSMGFR sequence) when they are in dimer form; NME7 has two (2) binding sites for MUC1^{∗} receptor extra cellular domain and also dimerizes the receptor. NME1 dimers, NME6 dimers and NME7 are the preferred NME family members for use as MUC1^{∗} ligands to induce or maintain cells in a less mature state than the starting cells. Other NME family members that are able to bind to and dimerize the MUC1^{∗} receptor are also contemplated for use as MUC1* ligands to induce or maintain cells in a less mature state than the starting cells.

As used herein, "pluripotency markers" are those genes and proteins whose expression is increased when cells revert to a less mature state than the starting cells. Pluripotency markers include OCT4, SOX2, NANOG, KLF4, KLF2, Tra 1-60, Tra 1-81, SSEA4, and REX-1 as well as others previously described and those currently being discovered. For example, fibroblast cells express no detectable or low levels of these pluripotency markers, but express a fibroblast differentiation marker called CD13. To determine if a cell is becoming less mature than the starting cells, one could measure a difference in the expression levels of the pluripotency markers between the starting cells and the resultant cells.

As used herein, "primed stem cells" are cells that resemble and share traits and characteristics of cells from the epiblast portion of a blastocyst.

As used herein, the term "specifically binds" refers to a non-random binding reaction between two molecules, for example between an antibody molecule immunoreacting with an antigen, or a non-antibody ligand reacting with another polypeptide, such as NM23 specifically binding with MUC1^{∗} or an antibody binding to MUC1^{∗} or a cleaving enzyme binding to MUC1 or MUC1^{∗}.

As used herein, "pluripotent" stem cell refers to stem cells that can differentiate to all three germlines, endoderm, ectoderm and mesoderm, to differentiate into any cell type in the body, but cannot give rise to a complete organism. A totipotent stem cell is one that can differentiate or mature into a complete organism such as a human being. With reference to embryonic pluripotent stem cells, they are cells derived from the inner cell mass of a blastocyst. Typical markers of pluripotency are OCT4, KLF4, NANOG, Tra 1-60, Tra 1-81 and SSEA4.

As used herein, "multipotent" stem cells refer to stem cells that can differentiate into other cell types wherein the number of different cell types is limited.

As used herein, "semi-pluripotent" or "pre-iPS state" refers to a cell that has some or all of the morphological characteristics of a pluripotent stem cell, but its level of expression of the pluripotency markers or its ability to differentiate to all three germlines is less than that of a pluripotent stem cell.

As used herein, "stem-like" morphology refers to a morphology that resembles that of a stem cell, a level of expression of one or more of the pluripotency genes, or an ability to differentiate into multiple cell types. Stem-like morphology is when the cells have a rounded shape, and are rather small compared to the size of their nucleus, which is often has a large nucleus to cytoplasm ratio, which is characteristic of pluripotent stem cells. By contrast, fibroblast morphology is when cells have a long, spindly shape and do not have a large nucleus to cytoplasm ratio. Additionally, pluripotent stem cells are non-adherent, whereas other cell types, such as fibroblasts, are adherent.

As used herein, "vector", "polynucleotide vector", "construct" and "polynucleotide construct" are used interchangeably herein. A polynucleotide vector of this invention may be in any of several forms, including, but not limited to, RNA, DNA, RNA encapsulated in a retroviral coat, DNA encapsulated in an adenovirus coat, DNA packaged in another viral or viral-like form (such as herpes simplex, and adeno-associated virus (AAV)), DNA encapsulated in liposomes, DNA complexed with polylysine, complexed with synthetic polycationic molecules, complexed with compounds such as polyethylene glycol (PEG) to immunologically "mask" the molecule and/or increase half-life, or conjugated to a non-viral protein. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

As used herein, "multimer" refers to a plurality of monomers that are covalently linked together or non-covalently fused to each other.

As used herein, "higher order multimer" refers to a plurality of monomers that are covalently linked together or non-covalently fused to each other, which is greater than a dimer.

### Sequence Listing Free Text

As regards the use of nucleotide symbols other than a, g, c, t, they follow the convention set forth in WIPO Standard ST.25, Appendix 2, Table 1, wherein k represents t or g; n represents a, c, t or g; m represents a or c; r represents a or g; s represents c or g; w represents a or t and y represents c or t.

MTPGTQSPFF LLLLLTVLTV VTGSGHASST PGGEKETSAT QRSSVPSSTE
KNAVSMTSSV LSSHSPGSGS STTQGQDVTL APATEPASGS AATWGQDVTS
VPVTRPALGS TTPPAHDVTS APDNKPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS
TAPPAHGVTS APDTRPAPGS TAPPAHGVTS APDTRPAPGS TAPPAHGVTS
APDTRPAPGS TAPPAHGVTS APDTRPAPGS TAPPAHGVTS APDNRPALGS
TAPPVHNVTS ASGSASGSAS TLVHNGTSAR ATTTPASKST PFSIPSHHSD
TPTTLASHST KTDASSTHHS SVPPLTSSNH STSPQLSTGV SFFFLSFHIS
NLQFNSSLED PSTDYYQELQ RDISEMFLQI YKQGGFLGLS NIKFRPGSVV
VQLTLAFREG TINVHDVETQ FNQYKTEAAS RYNLTISDVS VSDVPFPFSA

QSGAGVPGWG IALLVLVCVL VALAIVYLIA LAVCQCRRKN YGQLDIFPAR
DTYHPMSEYP TYHTHGRYVP PSSTDRSPYE KVSAGNGGSS LSYTNPAVAA
ASANL (SEQ ID NO: 1) describes full-length MUC1 Receptor (Mucin 1 precursor, Genbank
Accession number: P15941).

MTPGTQSPFFLLLLLTVLT (SEQ ID NO: 2)
MTPGTQSPFFLLLLLTVLT WTA (SEQ ID NO: 3)
MTPGTQSPFFLLLLLTVLT WTG (SEQ ID NO: 4)
SEQ ID NOS:2, 3 and 4 describe N-terminal MUC-1 signaling sequence for directing MUC1 receptor and truncated isoforms to cell membrane surface. Up to 3 amino acid residues may be absent at C-terminal end as indicated by variants in SEQ ID NOS:2, 3 and 4. 
GTINVHDVETQFNQYKTEAASRYNLTISDVSVSDVPFPFSAQSGAGVPGWGIALLVLVCVLV
ALAIVYLIALAVCQCRRKNYGQLDIFPARDTYHPMSEYPTYHTHGRYVPPSSTDRSPYEKVS
AGNGGSSLSYTNPAVAAASANL (SEQ ID NO:5) describes a truncated MUC1 receptor
isoform having nat-PSMGFR at its N-terminus and including the transmembrane and
cytoplasmic sequences of a full-length MUC1 receptor.
GTINVHDVETQFNQYKTEAASRYNLTISDVSVSDVPFPFSAQSGA (SEQ ID NO:6) describes Native Primary Sequence of the MUC1 Growth Factor Receptor (nat-PSMGFR - an example of "PSMGFR"):
TINVHDVETQFNQYKTEAASRYNLTISDVSVSDVPFPFSAQSGA (SEQ ID NO:7) describes Native Primary Sequence of the MUC1 Growth Factor Receptor (nat-PSMGFR - An example of "PSMGFR"), having a single amino acid deletion at the N-terminus of SEQ ID NO:6).
GTINVHDVETQFNQYKTEAASPYNLTISDVSVSDVPFPFSAQSGA (SEQ ID NO:8) describes "SPY" functional variant of the native Primary Sequence of the MUC1 Growth Factor Receptor having enhanced stability (var-PSMGFR - An example of "PSMGFR").
TINVHDVETQFNQYKTEAASPYNLTISDVSVSDVPFPFSAQSGA (SEQ ID NO: 9) describes "SPY" functional variant of the native Primary Sequence of the MUC1 Growth Factor Receptor having enhanced stability (var-PSMGFR - An example of "PSMGFR"), having a single amino acid deletion at the C-terminus of SEQ ID NO:8).

tgtcagtgccgccgaaagaactacgggcagctggacatctttccagcccgggatacctacca
tcctatgagcgagtaccccacctaccacacccatgggcgctatgtgccccctagcagtaccg
atcgtagcccctatgagaaggtttctgcaggtaacggtggcagcagcctctcttacacaaac
ccagcagtggcagccgcttctgccaacttg (SEQ ID NO:10) describes MUC1 cytoplasmic
domain nucleotide sequence.

CQCRRKNYGQLDIFPARDTYHPMSEYPTYHTHGRYVPPSSTDRSPYEKVSAGNGGSSLSYTN
PAVAAASANL (SEQ ID NO:11) describes MUC1 cytoplasmic domain amino acid sequence.

### Human NM23 H1

(DNA) 
atggccaactgtgagcgtaccttcattgcgatcaaaccagatggggtccagcggggtcttgtgggagagattatcaag
cgttttgagcagaaaggattccgccttgttggtctgaaattcatgcaagcttccgaagatcttctcaaggaacactacgttgacctgaagg
 
accgtccattctttgccggcctggtgaaatacatgcactcagggccggtagttgccatggtctgggaggggctgaatgtggtgaagac
gggccgagtcatgctcggggagaccaaccctgcagactccaagcctgggaccatccgtggagacttctgcatacaagttggcagga
acattatacatggcagtgattctgtggagagtgcagagaaggagatcggcttgtggtttcaccctgaggaactggtagattacacgagc
tgtgctcagaactggatctatgaatga (SEQ ID NO:12)
(amino acids)

MANCERTFIAIKPDGVQRGLVGEIIKRFEQKGFRLVGLKFMQASEDLLKEH
YVDLKDRPFFAGLVKYMHSGPVVAMVWEGLNVVKTGRVMLGETNPADSKPGTIRG
DFCIQVGRNIIHGSDSVESAEKEIGLWFHPEELVDYTSCAQNWIYE- (SEQ ID NO:13)

### Mouse NM23 H1

(DNA) 
atggccaacagtgagcgcaccttcattgccatcaagcctgatggggtccagcgggggctggtgggcgagatcatca
agcggttcgagcagaaggggttccgccttgttggtctgaagtttctgcaggcttcagaggaccttctcaaggagcactacactgacctg
aaggaccgccccttctttactggcctggtgaaatacatgcactcaggaccagtggttgctatggtctgggagggtctgaatgtggtgaa
gacaggccgcgtgatgcttggagagaccaaccccgcagactctaagcctgggaccatacgaggagacttctgcatccaagttggca
ggaacatcattcatggcagcgattctgtaaagagcgcagagaaggagatcagcttgtggtttcagcctgaggagctggtggagtacaa
gagctgtgcgcagaactggatctatgagtga (SEQ ID NO:14)
(amino acids)

MANSERTFIAIKPDGVQRGLVGEIIKRFEQKGFRLVGLKFLQASEDLLKEH
YTDLKDRPFFTGLVKYMHSGPVVAMVWEGLNVVKTGRVMLGETNPADSKPGTIRG
DFCIQVGRNIIHGSDSVKSAEKEISLWFQPEELVEYKSCAQNWIYE- (SEQ ID NO:15)

### Human NM23 H2

(DNA) 
atggccaacctggagcgcaccttcatcgccatcaagccggacggcgtgcagcgcggcctggtgggcgagatcatc
aagcgcttcgagcagaagggattccgcctcgtggccatgaagttcctccgggcctctgaagaacacctgaagcagcactacattgac
ctgaaagaccgaccattcttccctgggctggtgaagtacatgaactcagggccggttgtggccatggtctgggaggggctgaacgtg
gtgaagacaggccgagtgatgcttggggagaccaatccagcagattcaaagccaggcaccattcgtggggacttctgcattcaggtt
ggcaggaacatcattcatggcagtgattcagtaaaaagtgctgaaaaagaaatcagcctatggtttaagcctgaagaactggttgacta
caagtcttgtgctcatgactgggtctatgaataa (SEQ ID NO:16)
(amino acids)

MANLERTFIAIKPDGVQRGLVGEIIKRFEQKGFRLVAMKFLRASEEHLKQH
YIDLKDRPFFPGLVKYMNSGPVVAMVWEGLNVVKTGRVMLGETNPADSKPGTIRG
DFCIQVGRNIIHGSDSVKSAEKEISLWFKPEELVDYKSCAHDWVYE- (SEQ ID NO:17)

### Mouse NM23 H2

(DNA) 
atggccaacctcgagcgtaccttcattgccatcaagccagatggcgtgcagcgcggcctggtgggcgagatcatca
aacggttcgagcagaaggggttccgcctggtggccatgaagttccttcgggcctctgaagaacacctgaagcagcattacatcgacct
gaaagaccgtcctttcttcccggggctggtgaagtacatgaactcggggcccgtggtggccatggtctgggaggggctcaatgtggt
gaaaacgggccgagtgatgctgggggagaccaatccagctgattcaaaaccaggcaccatccgtggggatttctgcattcaagttgg
caggaacatcattcatggcagtgattcagtggagagtgctgagaaagagatccatctgtggtttaagcccgaagaactgatcgactaca
agtcttgtgcccatgactgggtgtacgagtag (SEQ ID NO:18)
(amino acids)

MANLERTFIAIKPDGVQRGLVGEIIKRFEQKGFRLVAMKFLRASEEHLKQH
YIDLKDRPFFPGLVKYMNSGPVVAMVWEGLNVVKTGRVMLGETNPADSKPGTIRG
DFCIQVGRNIIHGSDSVESAEKEIHLWFKPEELIDYKSCAHDWVYE- (SEQ ID NO:19)

### Mouse NME6

(DNA) 
Atgacctccatcttgcgaagtccccaagctcttcagctcacactagccctgatcaagcctgatgcagttgcccaccca
ctgatcctggaggctgttcatcagcagattctgagcaacaagttcctcattgtacgaacgagggaactgcagtggaagctggaggact
gccggaggttttaccgagagcatgaagggcgttttttctatcagcggctggtggagttcatgacaagtgggccaatccgagcctatatc
cttgcccacaaagatgccatccaactttggaggacactgatgggacccaccagagtatttcgagcacgctatatagccccagattcaat
tcgtggaagtttgggcctcactgacacccgaaatactacccatggctcagactccgtggtttccgccagcagagagattgcagccttctt
ccctgacttcagtgaacagcgctggtatgaggaggaggaaccccagctgcggtgtggtcctgtgcactacagtccagaggaaggtat
ccactgtgcagctgaaacaggaggccacaaacaacctaacaaaacctag (SEQ ID NO:20)
(amino acids)

MTSILRSPQALQLTLALIKPDAVAHPLILEAVHQQILSNKFLIVRTRELQWK
LEDCRRFYREHEGRFFYQRLVEFMTSGPIRAYILAHKDAIQLWRTLMGPTRVFRARY
IAPDSIRGSLGLTDTRNTTHGSDSVVSASREIAAFFPDFSEQRWYEEEEPQLRCGPVHY
SPEEGIHCAAETGGHKQPNKT- (SEQ ID NO:21)

### Human NME6:

(DNA) 
Atgacccagaatctggggagtgagatggcctcaatcttgcgaagccctcaggctctccagctcactctagccctgat
caagcctgacgcagtcgcccatccactgattctggaggctgttcatcagcagattctaagcaacaagttcctgattgtacgaatgagag
aactactgtggagaaaggaagattgccagaggttttaccgagagcatgaagggcgttttttctatcagaggctggtggagttcatggcc
agcgggccaatccgagcctacatccttgcccacaaggatgccatccagctctggaggacgctcatgggacccaccagagtgttccga
gcacgccatgtggccccagattctatccgtgggagtttcggcctcactgacacccgcaacaccacccatggttcggactctgtggtttc
 
agccagcagagagattgcagccttcttccctgacttcagtgaacagcgctggtatgaggaggaagagccccagttgcgctgtggccct
gtgtgctatagcccagagggaggtgtccactatgtagctggaacaggaggcctaggaccagcctga (SEQ ID NO:22)
(amino acids)

MTQNLGSEMASILRSPQALQLTLALIKPDAVAHPLILEAVHQQILSNKFLIV
RMRELLWRKEDCQRFYREHEGRFFYQRLVEFMASGPIRAYILAHKDAIQLWRTLMG
PTRVFRARHVAPDSIRGSFGLTDTRNTTHGSDSVVSASREIAAFFPDFSEQRWYEEEE
PQLRCGPVCYSPEGGVHYVAGTGGLGPA- (SEQ ID NO:23)

### Human NME6 1:

(DNA) 
Atgacccagaatctggggagtgagatggcctcaatcttgcgaagccctcaggctctccagctcactctagccctgat
caagcctgacgcagtcgcccatccactgattctggaggctgttcatcagcagattctaagcaacaagttcctgattgtacgaatgagag
aactactgtggagaaaggaagattgccagaggttttaccgagagcatgaagggcgttttttctatcagaggctggtggagttcatggcc
agcgggccaatccgagcctacatccttgcccacaaggatgccatccagctctggaggacgctcatgggacccaccagagtgttccga
gcacgccatgtggccccagattctatccgtgggagtttcggcctcactgacacccgcaacaccacccatggttcggactctgtggtttc
agccagcagagagattgcagccttcttccctgacttcagtgaacagcgctggtatgaggaggaagagccccagttgcgctgtggccct
gtgtga (SEQ ID NO:24)
(amino acids)

MTQNLGSEMASILRSPQALQLTLALIKPDAVAHPLILEAVHQQILSNKFLIV
RMRELLWRKEDCQRFYREHEGRFFYQRLVEFMASGPIRAYILAHKDAIQLWRTLMG
PTRVFRARHVAPDSIRGSFGLTDTRNTTHGSDSVVSASREIAAFFPDFSEQRWYEEEE
PQLRCGPV- (SEQ ID NO:25)

### Human NME6 2:

(DNA) 
Atgctcactctagccctgatcaagcctgacgcagtcgcccatccactgattctggaggctgttcatcagcagattctaa
gcaacaagttcctgattgtacgaatgagagaactactgtggagaaaggaagattgccagaggttttaccgagagcatgaagggcgtttt
ttctatcagaggctggtggagttcatggccagcgggccaatccgagcctacatccttgcccacaaggatgccatccagctctggagga
cgctcatgggacccaccagagtgttccgagcacgccatgtggccccagattctatccgtgggagtttcggcctcactgacacccgcaa
caccacccatggttcggactctgtggtttcagccagcagagagattgcagccttcttccctgacttcagtgaacagcgctggtatgagg
aggaagagccccagttgcgctgtggccctgtgtga (SEQ ID NO:26)
(amino acids)

MLTLALIKPDAVAHPLILEAVHQQILSNKFLIVRMRELLWRKEDCQRFYRE
HEGRFFYQRLVEFMASGPIRAYILAHKDAIQLWRTLMGPTRVFRARHVAPDSIRGSF

GLTDTRNTTHGSDSVVSASREIAAFFPDFSEQRWYEEEEPQLRCGPV- (SEQ ID
NO:27)

### Human NME6 3:

(DNA) 
Atgctcactctagccctgatcaagcctgacgcagtcgcccatccactgattctggaggctgttcatcagcagattctaa
gcaacaagttcctgattgtacgaatgagagaactactgtggagaaaggaagattgccagaggttttaccgagagcatgaagggcgtttt
ttctatcagaggctggtggagttcatggccagcgggccaatccgagcctacatccttgcccacaaggatgccatccagctctggagga
cgctcatgggacccaccagagtgttccgagcacgccatgtggccccagattctatccgtgggagtttcggcctcactgacacccgcaa
caccacccatggttcggactctgtggtttcagccagcagagagattgcagccttcttccctgacttcagtgaacagcgctggtatgagg
aggaagagccccagttgcgctgtggccctgtgtgctatagcccagagggaggtgtccactatgtagctggaacaggaggcctagga
ccagcctga (SEQ ID NO:28)
(amino acids)

MLTLALIKPDAVAHPLILEAVHQQILSNKFLIVRMRELLWRKEDCQRFYRE
HEGRFFYQRLVEFMASGPIRAYILAHKDAIQLWRTLMGPTRVFRARHVAPDSIRGSF
GLTDTRNTTHGSDSVVSASREIAAFFPDFSEQRWYEEEEPQLRCGPVCYSPEGGVHY
VAGTGGLGPA- (SEQ ID NO:29)

### Human NM23-H7-1

(DNA) 
atgaatcatagtgaaagattcgttttcattgcagagtggtatgatccaaatgcttcacttcttcgacgttatgagcttttatttt
acccaggggatggatctgttgaaatgcatgatgtaaagaatcatcgcacctttttaaagcggaccaaatatgataacctgcacttggaag
atttatttataggcaacaaagtgaatgtcttttctcgacaactggtattaattgactatggggatcaatatacagctcgccagctgggcagta
ggaaagaaaaaacgctagccctaattaaaccagatgcaatatcaaaggctggagaaataattgaaataataaacaaagctggatttact
ataaccaaactcaaaatgatgatgctttcaaggaaagaagcattggattttcatgtagatcaccagtcaagaccctttttcaatgagctgat
ccagtttattacaactggtcctattattgccatggagattttaagagatgatgctatatgtgaatggaaaagactgctgggacctgcaaact
ctggagtggcacgcacagatgcttctgaaagcattagagccctctttggaacagatggcataagaaatgcagcgcatggccctgattct
tttgcttctgcggccagagaaatggagttgttttttccttcaagtggaggttgtgggccggcaaacactgctaaatttactaattgtacctgtt
gcattgttaaaccccatgctgtcagtgaaggactgttgggaaagatcctgatggctatccgagatgcaggttttgaaatctcagctatgca
gatgttcaatatggatcgggttaatgttgaggaattctatgaagtttataaaggagtagtgaccgaatatcatgacatggtgacagaaatgt
attctggcccttgtgtagcaatggagattcaacagaataatgctacaaagacatttcgagaattttgtggacctgctgatcctgaaattgcc
cggcatttacgccctggaactctcagagcaatctttggtaaaactaagatccagaatgctgttcactgtactgatctgccagaggatggc
ctattagaggttcaatacttcttcaagatcttggataattag (SEQ ID NO:30)
(amino acids)

MNHSERFVFIAEWYDPNASLLRRYELLFYPGDGSVEMHDVKNHRTFLKR
TKYDNLHLEDLFIGNKVNVFSRQLVLIDYGDQYTARQLGSRKEKTLALIKPDAISKA
GEIIEIINKAGFTITKLKMMMLSRKEALDFHVDHQSRPFFNELIQFITTGPIIAMEILRDD
AICEWKRLLGPANSGVARTDASESIRALFGTDGIRNAAHGPDSFASAAREMELFFPSS
GGCGPANTAKFTNCTCCIVKPHAVSEGLLGKILMAIRDAGFEISAMQMFNMDRVNV
EEFYEVYKGVVTEYHDMVTEMYSGPCVAMEIQQNNATKTFREFCGPADPEIARHLR
PGTLRAIFGKTKIQNAVHCTDLPEDGLLEVQYFFKILDN- (SEQ ID NO:31)

### Human NM23-H7-1 sequence optimized for E. coli expression

(DNA) 
atgaatcactccgaacgctttgtttttatcgccgaatggtatgacccgaatgcttccctgctgcgccgctacgaactgct
gttttatccgggcgatggtagcgtggaaatgcatgacgttaaaaatcaccgtacctttctgaaacgcacgaaatatgataatctgcatctg
gaagacctgtttattggcaacaaagtcaatgtgttctctcgtcagctggtgctgatcgattatggcgaccagtacaccgcgcgtcaactg
ggtagtcgcaaagaaaaaacgctggccctgattaaaccggatgcaatctccaaagctggcgaaattatcgaaattatcaacaaagcgg
gtttcaccatcacgaaactgaaaatgatgatgctgagccgtaaagaagccctggattttcatgtcgaccaccagtctcgcccgtttttcaa
tgaactgattcaattcatcaccacgggtccgattatcgcaatggaaattctgcgtgatgacgctatctgcgaatggaaacgcctgctggg
cccggcaaactcaggtgttgcgcgtaccgatgccagtgaatccattcgcgctctgtttggcaccgatggtatccgtaatgcagcacatg
gtccggactcattcgcatcggcagctcgtgaaatggaactgtttttcccgagctctggcggttgcggtccggcaaacaccgccaaattt
accaattgtacgtgctgtattgtcaaaccgcacgcagtgtcagaaggcctgctgggtaaaattctgatggcaatccgtgatgctggcttt
gaaatctcggccatgcagatgttcaacatggaccgcgttaacgtcgaagaattctacgaagtttacaaaggcgtggttaccgaatatca
cgatatggttacggaaatgtactccggtccgtgcgtcgcgatggaaattcagcaaaacaatgccaccaaaacgtttcgtgaattctgtgg
tccggcagatccggaaatcgcacgtcatctgcgtccgggtaccctgcgcgcaatttttggtaaaacgaaaatccagaacgctgtgcact
gtaccgatctgccggaagacggtctgctggaagttcaatactttttcaaaattctggataattag (SEQ ID NO:32)
(amino acids)

MNHSERFVFIAEWYDPNASLLRRYELLFYPGDGSVEMHDVKNHRTFLKR
TKYDNLHLEDLFIGNKVNVFSRQLVLIDYGDQYTARQLGSRKEKTLALIKPDAISKA
GEIIEIINKAGFTITKLKMMMLSRKEALDFHVDHQSRPFFNELIQFITTGPIIAMEILRDD
AICEWKRLLGPANSGVARTDASESIRALFGTDGIRNAAHGPDSFASAAREMELFFPSS
GGCGPANTAKFTNCTCCIVKPHAVSEGLLGKILMAIRDAGFEISAMQMFNMDRVNV
EEFYEVYKGVVTEYHDMVTEMYSGPCVAMEIQQNNATKTFREFCGPADPEIARHLR
PGTLRAIFGKTKIQNAVHCTDLPEDGLLEVQYFFKILDN- (SEQ ID NO:33)

### Human NM23-H7-2

(DNA) 
atgcatgatgtaaagaatcatcgcacctttttaaagcggaccaaatatgataacctgcacttggaagatttatttataggc
aacaaagtgaatgtcttttctcgacaactggtattaattgactatggggatcaatatacagctcgccagctgggcagtaggaaagaaaaa
acgctagccctaattaaaccagatgcaatatcaaaggctggagaaataattgaaataataaacaaagctggatttactataaccaaactc
aaaatgatgatgctttcaaggaaagaagcattggattttcatgtagatcaccagtcaagaccctttttcaatgagctgatccagtttattaca
actggtcctattattgccatggagattttaagagatgatgctatatgtgaatggaaaagactgctgggacctgcaaactctggagtggca
cgcacagatgcttctgaaagcattagagccctctttggaacagatggcataagaaatgcagcgcatggccctgattcttttgcttctgcg
gccagagaaatggagttgttttttccttcaagtggaggttgtgggccggcaaacactgctaaatttactaattgtacctgttgcattgttaaa
ccccatgctgtcagtgaaggactgttgggaaagatcctgatggctatccgagatgcaggttttgaaatctcagctatgcagatgttcaata
tggatcgggttaatgttgaggaattctatgaagtttataaaggagtagtgaccgaatatcatgacatggtgacagaaatgtattctggccct
tgtgtagcaatggagattcaacagaataatgctacaaagacatttcgagaattttgtggacctgctgatcctgaaattgcccggcatttac
gccctggaactctcagagcaatctttggtaaaactaagatccagaatgctgttcactgtactgatctgccagaggatggcctattagagg
ttcaatacttcttcaagatcttggataattga (SEQ ID NO:34)
(amino acids)

MHDVKNHRTFLKRTKYDNLHLEDLFIGNKVNVFSRQLVLIDYGDQYTAR
QLGSRKEKTLALIKPDAISKAGEIIEIINKAGFTITKLKMMMLSRKEALDFHVDHQSRP
FFNELIQFITTGPIIAMEILRDDAICEWKRLLGPANSGVARTDASESIRALFGTDGIRNA
AHGPDSFASAAREMELFFPSSGGCGPANTAKFTNCTCCIVKPHAVSEGLLGKILMAIR
DAGFEISAMQMFNMDRVNVEEFYEVYKGVVTEYHDMVTEMYSGPCVAMEIQQNN
ATKTFREFCGPADPEIARHLRPGTLRAIFGKTKIQNAVHCTDLPEDGLLEVQYFFKILD
N- (SEQ ID NO:35)

### Mouse NM23-H7-1

(DNA) 
atgagagcctgtcagcagggaagaagttccagtttggtttctccatatatggcacccaagaatcagagcgagagattc
gctttcattgcagagtggtatgatccaaatgcttcattgctccgacgctatgagctgcttttaccccacagacggatctgttgaaatgca
tgatgtaaagaatcgtcgcaccttcttaaagcggaccaagtatgaggacctgcgcctggaagatctatttataggcaacaaagtcaatgt
gttttctcgacagctggtgttgattgactatggggaccaatacacagcccgccagctgggcagcaggaaagagaaaactttagccctg
atcaaaccagatgcagtgtcaaaggccggagaaatcattgagatgataaacaaaagtggatttactataaccaaactccgaatgatgac
tctgacaaggaaagaagcagcggactttcatgtagaccatcactcaagacctttttataacgaactgatccagtttatcacaagtgggcct
gttattgccatggagatcttaagagatgacgcgatctgtgagtggaaaaggttgcttggacccgcaaactctgggctatcacggacaga
tgcccccggaagcatccgagccctctttgggacagatggcgtgagaaatgcagctcacggccctgatacttttgcatctgctgccaga
gaaatggaattgttttttccttcaagtggaggctgtgggccagcgaacactgctaaatttaccaattgcacctgttgcatcattaagcctcat
gctatcagtgaaggaatgttgggaaagattttaatagctattcgggatgcatgctttggaatgtcagcgatacagatgttcaatttggatcg
ggctaatgttgaagaattctatgaagtctataaaggtgtagtgtctgagtataatgatatggtgacagagctgtgctccggcccttgcgta
 
gcaatagagatccaacagagcaaccctacaaagacatttcgagaattctgcggacctgctgatcctgaaatcgcccggcatttacgac
ctgagaccctcagggcaatttttggtaaaactaaggttcaaaatgctgttcattgcacggatctgccggaggatgggctcctggaggtcc
agtatttcttcaagatcttggataattag (SEQ ID NO:36)
(amino acids)

MRACQQGRSSSLVSPYMAPKNQSERFAFIAEWYDPNASLLRRYELLFYPT
DGSVEMHDVKNRRTFLKRTKYEDLRLEDLFIGNKVNVFSRQLVLIDYGDQYTARQL
GSRKEKTLALIKPDAVSKAGEIIEMINKSGFTITKLRMMTLTRKEAADFHVDHHSRPF
YNELIQFITSGPVIAMEILRDDAICEWKRLLGPANSGLSRTDAPGSIRALFGTDGVRNA
AHGPDTFASAAREMELFFPSSGGCGPANTAKFTNCTCCIIKPHAISEGMLGKILIAIRD
ACFGMSAIQMFNLDRANVEEFYEVYKGVVSEYNDMVTELCSGPCVAIEIQQSNPTKT
FREFCGPADPEIARHLRPETLRAIFGKTKVQNAVHCTDLPEDGLLEVQYFFKILDN-
(SEQ ID NO:37)

### Mouse NM23-H7-2

(DNA) 
atgagagcctgtcagcagggaagaagttccagtttggtttctccatatatggcacccaagaatcagagcgagagattc
gctttcattgcagagtggtatgatccaaatgcttcattgctccgacgctatgagctgctgttttaccccacagacggatctgttgaaatgca
tgatgtaaagaatcgtcgcaccttcttaaagcggaccaagtatgaggacctgcgcctggaagatctatttataggcaacaaagtcaatgt
gttttctcgacagctggtgttgattgactatggggaccaatacacagcccgccagctgggcagcaggaaagagaaaactttagccctg
atcaaaccagatgcagtgtcaaaggccggagaaatcattgagatgataaacaaaagtggatttactataaccaaactccgaatgatgac
tctgacaaggaaagaagcagcggactttcatgtagaccatcactcaagacctttttataacgaactgatccagtttatcacaagtgggcct
gttattgccatggagatcttaagagatgacgcgatctgtgagtggaaaaggttgcttggacccgcaaactctgggctatcacggacaga
tgcccccggaagcatccgagccctctttgggacagatggcgtgagaaatgcagctcacggccctgatacttttgcatctgctgccaga
gaaatggaattgttttttccttcaagtggaggctgtgggccagcgaacactgctaaatttaccaattgcacctgttgcatcattaagcctcat
gctatcagtgaagatttatttattcattatatgtaa (SEQ ID NO:38)
(amino acids)

MRACQQGRSSSLVSPYMAPKNQSERFAFIAEWYDPNASLLRRYELLFYPT
DGSVEMHDVKNRRTFLKRTKYEDLRLEDLFIGNKVNVFSRQLVLIDYGDQYTARQL
GSRKEKTLALIKPDAVSKAGEIIEMINKSGFTITKLRMMTLTRKEAADFHVDHHSRPF
YNELIQFITSGPVIAMEILRDDAICEWKRLLGPANSGLSRTDAPGSIRALFGTDGVRNA
AHGPDTFASAAREMELFFPSSGGCGPANTAKFTNCTCCIIKPHAISEDLFIHYM- (SEQ
ID NO:39)

### Mouse NME8 variant1

(DNA) 
atggcaagcaaaaagcgtgaagtccagctacagtcagtcgtcaatagtcagaacttgtgggatgagatgttgctgaac
aaaggcttaacagtgattgatgtttaccaagcctggtgtggaccttgcaaagccgtgcaaagtttattcagaaaactaaaaaatgaactg
aacgaagatgagattcttcacttcgtcgttgctgaagctgacaacattgtgactctccagccatttagagataaatgtgagcccgtgtttct
ctttagtcttaatggtaaaatcattgcaaagattcagggtgcaaatgctccacttatcaatagaaaagtcattaccttgatagatgaagaga
ggaaaattgtagcaggtgaaatggatcgtcctcagtatgttgaaattccactagtagatgcaatcgatgaagaatatggggaagtacagt
atgaaagtgctgcggaagtttacaatatggcaattatcaaacctgatgctgtactcatgagaaaaaatatagaagttagggaaaaaatag
ccaaagaaggatttgttatagaaatacaagaaaacctgattctccctgaagaggtagtgagggaattctacactcatatagcagaccag
cctgactttgaagagtttgtcgtttctatgacaaatggcctcagctgtgtgctcattgtatctcaagaagactccgaggttattcaggaaga
aactctcccgcagactgatacagaagaagaacctggcgttttggaagagcctcacgttaggtttgcacctgtgatgataaagaagaaac
gggacagtttgcaagagtatatggaccgacagcatatgtctgattactgcgatgtcgaggacgatgcggttaaggtctctaagctcattg
acatattattccctgattttaaaactatgaaaagcacgaatgtacaaacgacgctagcattactgcatccagacatctgtgaggaagaga
aagatgacgtgttgaacgttattcacaatgaagggttcaccatactgatgcagaggcaaatcgtattatcagaggaagaagcaagaaca
gtgtgcaagatccatgaaaacgaagagtattttgataatcttatagggcacatgaccagtaatcactcttatgtccttgctctacggaggg
aaaatggtgtggaatattggaaaacattaattgggccaaaaacgattgaggaagcttatgcatctcatccacagagtttatgtgtacagttt
gcttcagggaattttcctaccaaccagttctacgggagcagttcaaaagcagcagctgagaaggaaatagcgcatttcttccctcccca
gagcacacttgcattgatcaagcctcatgtgacacacaaagaaagaatggagatcctgaagaccattaaagaggcaggatttgagctg
accctgatgaaggaaatgcacctgactccagagcatgcaaacaaaatttatttcaaaataacaggaaaagatttttataaaaatgtattgg
aagtcttatctttgggcatgtcgctagtcatggttttgaccaagtggaatgctgttgcagaatggaggcgaatggttggcccagtagacc
cagaagaagcaaaactgctctccccagaatccctccgagccaaatatggactagacattttgagaaatgctgtccatggggcgtctaac
ttttctgaagcatcagaaatcattagtaatgtgttcacagagggtaatcctgagaactag (SEQ ID NO:40)
(amino acids)

MASKKREVQLQSVVNSQNLWDEMLLNKGLTVIDVYQAWCGPCKAVQSL
FRKLKNELNEDEILHFVVAEADNIVTLQPFRDKCEPVFLFSLNGKIIAKIQGANAPLIN
RKVITLIDEERKIVAGEMDRPQYVEIPLVDAIDEEYGEVQYESAAEVYNMAIIKPDAV
LMRKNIEVREKIAKEGFVIEIQENLILPEEVVREFYTHIADQPDFEEFVVSMTNGLSCV
LIVSQEDSEVIQEETLPQTDTEEEPGVLEEPHVRFAPVMIKKKRDSLQEYMDRQHMS
DYCDVEDDAVKVSKLIDILFPDFKTMKSTNVQTTLALLHPDICEEEKDDVLNVIHNE
GFTILMQRQIVLSEEEARTVCKIHENEEYFDNLIGHMTSNHSYVLALRRENGVEYWK
TLIGPKTIEEAYASHPQSLCVQFASGNFPTNQFYGSSSKAAAEKEIAHFFPPQSTLALIK
PHVTHKERMEILKTIKEAGFELTLMKEMHLTPEHANKIYFKITGKDFYKNVLEVLSL
GMSLVMVLTKWNAVAEWRRMVGPVDPEEAKLLSPESLRAKYGLDILRNAVHGASN
FSEASEIISNVFTEGNPEN* (SEQ ID NO:41)

### Mouse NME8 variant2

(DNA) 
atggcaagcaaaaagcgtgaagtccagctacagtcagtcgtcaatagtcagaacttgtgggatgagatgttgctgaac
aaaggcttaacagtgattgatgtttaccaagcctggtgtggaccttgcaaagccgtgcaaagtttattcagaaaactaaaaaatgaactg
aacgaagatgagattcttcacttcgtcgttgctgaagctgacaacattgtgactctccagccatttagagataaatgtgagcccgtgtttct
ctttagtcttaatggtaaaatcattgcaaagattcagggtgcaaatgctccacttatcaatagaaaagtcattaccttgatagatgaagaga
ggaaaattgtagcaggtgaaatggatcgtcctcagtatgttgaaattccactagtagatgcaatcgatgaagaatatggggaagtacagt
atgaaagtgctgcggaagtttacaatatggcaattatcaaacctgatgctgtactcatgagaaaaaatatagaagttagggaaaaaatag
ccaaagaaggatttgttatagaaatacaagaaaacctgattctccctgaagaggtagtgagggaattctacactcatatagcagaccag
cctgactttgaagagtttgtcgtttctatgacaaatggcctcagctgtgtgctcattgtatctcaagaagactccgaggttattcaggaaga
aactctcccgcagactgatacagaagaagaacctggcgttttggaagagcctcacgttaggtttgcacctgtgatgataaagaagaaac
gggacagtttgcaagagtatatggaccgacagcatatgtctgattactgcgatgtcgaggacgatgcggttaaggtctctaagctcattg
acatattattccctgattttaaaactatgaaaagcacgaatgtacaaacgacgctagcattactgcatccagacatctgtgaggaagaga
aagatgacgtgttgaacgttattcacaatgaagggttcaccatactgatgcagaggcaaatcgtattatcagaggaagaagcaagaaca
gtgtgcaagatccatgaaaacgaagagtattttgataatcttatagggcacatgaccagtaatcactcttatgtccttgctctacggaggg
aaaatggtgtggaatattggaaaacattaattgggccaaaaacgattgaggaagcttatgcatctcatccacagagtttatgtgtacagttt
gcttcagggaattttcctaccaaccagttctacgggagcagttcaaaagcagcagctgagaaggaaatagcgcatttcttccctcccca
gagcacacttgcattgatcaagcctcatgtgacacacaaagaaagaattcacagaagctcaaggaggtaatga (SEQ ID
NO:42)
(amino acids)

MASKKREVQLQSVVNSQNLWDEMLLNKGLTVIDVYQAWCGPCKAVQSL
FRKLKNELNEDEILHFVVAEADNIVTLQPFRDKCEPVFLFSLNGKIIAKIQGANAPLIN
RKVITLIDEERKIVAGEMDRPQYVEIPLVDAIDEEYGEVQYESAAEVYNMAIIKPDAV
LMRKNIEVREKIAKEGFVIEIQENLILPEEVVREFYTHIADQPDFEEFVVSMTNGLSCV
LIVSQEDSEVIQEETLPQTDTEEEPGVLEEPHVRFAPVMIKKKRDSLQEYMDRQHMS
DYCDVEDDAVKVSKLIDILFPDFKTMKSTNVQTTLALLHPDICEEEKDDVLNVIHNE
GFTILMQRQIVLSEEEARTVCKIHENEEYFDNLIGHMTSNHSYVLALRRENGVEYWK
TLIGPKTIEEAYASHPQSLCVQFASGNFPTNQFYGSSSKAAAEKEIAHFFPPQSTLALIK
PHVTHKERIHRSSRR** (SEQ ID NO:43)

### Human NME8

(DNA) 
atggcaagcaaaaaacgagaagtccagttacagacagtcatcaataatcaaagcctgtgggatgagatgttgcagaa
caaaggcttaacagtgattgatgtttaccaagcctggtgtggaccttgcagagcaatgcaacctttattcagaaaattgaaaaatgaactg
aacgaagacgaaattctgcattttgctgtcgcagaagctgacaacattgtgactttgcagccatttagagataaatgtgaacctgtttttctc
 
tttagtgttaatggcaaaattatcgaaaagattcagggtgcaaatgcaccgcttgttaataaaaaagttattaatttgatcgatgaggagag
aaaaattgcagcaggtgaaatggctcgacctcagtatcctgaaattccattagtagactcagattcagaagttagtgaagaatcaccatg
tgaaagtgttcaggaattatacagtattgctattatcaaaccggatgctgtgattagtaaaaaagttctagaaattaaaagaaaaattacca
aagctggatttattatagaagcagagcataagacagtgctcactgaagaacaagttgtcaacttctatagtcgaatagcagaccagcgt
gacttcgaagagtttgtctcttttatgacaagtggcttaagctatattctagttgtatctcaaggaagtaaacacaatcctccctctgaagaa
accgaaccacagactgacaccgaacctaacgaacgatctgaggatcaacctgaggtcgaagcccaggttacacctggaatgatgaa
gaacaaacaagacagtttacaagaatatctggaaagacaacatttagctcagctctgtgacattgaagaggatgcagctaatgttgctaa
gttcatggatgctttcttccccgattttaaaaaaatgaaaagcatgaaattagaaaagacattggcattacttcgaccaaatctctttcatga
aaggaaagatgatgttttgcgtattattaaagatgaagacttcaaaatactggagcaaagacaagtagtattatcggaaaaagaagcaca
agcactgtgcaaggaatatgaaaatgaagactattttaataaacttatagaaaacatgaccagtggtccatctctagcccttgttttattgag
agacaatggcttgcaatactggaaacaattactgggaccaagaactgttgaagaagccattgaatattttccagagagtttatgtgcaca
gtttgcgatggacagtttgccggtcaaccagttgtatggcagcgattcattagaaaccgctgaaagggaaatacagcatttctttcctcttc
aaagcactttaggcttgattaaacctcatgcaacaagtgaacaaagagagcagatcctgaagatagttaaggaggctggatttgatctg
acacaggtgaagaaaatgttcctaactcctgagcaaacggagaaaatttatccaaaagtaacaggaaaagacttttataaagatttattg
gaaatgttatctgtgggtccatctatggtcatgattctgaccaagtggaatgctgttgcagaatggagacgattgatgggcccaacagac
ccagaagaagcaaaattactttcccctgactccatccgagcccagtttggaataagtaaattgaaaaacattgtccatggagcatctaac
gcctatgaagcaaaagaggttgttaatagactctttgaggatcctgaggaaaactaa (SEQ ID NO:44)
(amino acids)

MASKKREVQLQTVINNQSLWDEMLQNKGLTVIDVYQAWCGPCRAMQPL
FRKLKNELNEDEILHFAVAEADNIVTLQPFRDKCEPVFLFSVNGKIIEKIQGANAPLVN
KKVINLIDEERKIAAGEMARPQYPEIPLVDSDSEVSEESPCESVQELYSIAIIKPDAVISK
KVLEIKRKITKAGFIIEAEHKTVLTEEQVVNFYSRIADQRDFEEFVSFMTSGLSYILVV
SQGSKHNPPSEETEPQTDTEPNERSEDQPEVEAQVTPGMMKNKQDSLQEYLERQHL
AQLCDIEEDAANVAKFMDAFFPDFKKMKSMKLEKTLALLRPNLFHERKDDVLRIIKD
EDFKILEQRQVVLSEKEAQALCKEYENEDYFNKLIENMTSGPSLALVLLRDNGLQYW
KQLLGPRTVEEAIEYFPESLCAQFAMDSLPVNQLYGSDSLETAEREIQHFFPLQSTLGL
IKPHATSEQREQILKIVKEAGFDLTQVKKMFLTPEQTEKIYPKVTGKDFYKDLLEMLS
VGPSMVMILTKWNAVAEWRRLMGPTDPEEAKLLSPDSIRAQFGISKLKNIVHGASNA
YEAKEVVNRLFEDPEEN* (SEQ ID NO:45)

### NME8 E. coli optimized

(DNA) 
atgctggtcaataaaaaagtcatcaacctgatcgacgaagaacgcaaaatcgccgctggtgaaatggcacgcccgc
aatacccggaaatcccgctggttgatagcgactctgaagtttcagaagaatcgccgtgcgaatcagtgcaggaactgtattcgatcgca
 
attatcaaaccggatgctgtcatttccaaaaaagtgctggaaatcaaacgtaaaatcaccaaagcgggtttcattatcgaagccgaacat
aaaaccgtgctgacggaagaacaggtggttaatttttattcacgtatcgcggatcagcgcgactttgaagaatttgtttcgtttatgaccag
cggcctgtcttacattctggtcgtgagtcagggttccaaacacaatccgccgagcgaagaaacggaaccgcagaccgatacggaacc
gaacgaacgttctgaagaccagccggaagtggaagcacaagttaccccgggcatgatgaaaaataaacaggatagtctgcaagaat
acctggaacgccagcatctggctcaactgtgtgatatcgaagaagacgcggccaacgtggcgaaattcatggatgcctttttcccgga
cttcaagaaaatgaaaagcatgaaactggaaaaaaccctggccctgctgcgtccgaacctgttccacgaacgtaaagatgacgttctg
cgcatcatcaaagatgaagacttcaaaatcctggaacagcgccaagttgtcctgtctgaaaaagaagcacaggctctgtgcaaagaat
acgaaaacgaagattacttcaacaaactgatcgaaaacatgacctcaggtccgtcgctggcactggttctgctgcgtgataatggcctg
cagtattggaaacaactgctgggtccgcgcacggtcgaagaagccattgaatacttcccggaaagcctgtgtgcacagtttgctatgga
ttctctgccggtgaaccaactgtatggcagtgactccctggaaaccgcggaacgtgaaatccagcatttctttccgctgcaaagtaccct
gggtctgattaaaccgcacgcgacgtccgaacagcgcgaacaaattctgaaaatcgtcaaagaagccggcttcgatctgacccaggt
gaagaaaatgtttctgaccccggaacaaacggaaaaaatctatccgaaagtcacgggcaaagatttctacaaagacctgctggaaatg
ctgagtgttggtccgtccatggtcatgattctgaccaaatggaatgcggttgcagaatggcgtcgcctgatgggtccgacggatccgga
agaagcaaaactgctgagcccggactctattcgcgctcagtttggcatcagcaaactgaaaaacattgttcatggtgcgtccaatgcgt
atgaagcgaaagaagttgtgaaccgcctgtttgaagacccggaagaaaattaa (SEQ ID NO:46)
(amino acids)

MLVNKKVINLIDEERKIAAGEMARPQYPEIPLVDSDSEVSEESPCESVQEL
YSIAIIKPDAVISKKVLEIKRKITKAGFIIEAEHKTVLTEEQVVNFYSRIADQRDFEEFVS
FMTSGLSYILVVSQGSKHNPPSEETEPQTDTEPNERSEDQPEVEAQVTPGMMKNKQD
SLQEYLERQHLAQLCDIEEDAANVAKFMDAFFPDFKKMKSMKLEKTLALLRPNLFH
ERKDDVLRIIKDEDFKILEQRQVVLSEKEAQALCKEYENEDYFNKLIENMTSGPSLAL
VLLRDNGLQYWKQLLGPRTVEEAIEYFPESLCAQFAMDSLPVNQLYGSDSLETAERE
IQHFFPLQSTLGLIKPHATSEQREQILKIVKEAGFDLTQVKKMFLTPEQTEKIYPKVTG
KDFYKDLLEMLSVGPSMVMILTKWNAVAEWRRLMGPTDPEEAKLLSPDSIRAQFGI
SKLKNIVHGASNAYEAKEVVNRLFEDPEEN* (SEQ ID NO:47)

### NME8 1-2 E. coli optimized

(DNA) 
Atgctggtcaataaaaaagtcatcaacctgatcgacgaagaacgcaaaatcgccgctggtgaaatggcacgcccgc
aatacccggaaatcccgctggttgatagcgactctgaagtttcagaagaatcgccgtgcgaatcagtgcaggaactgtattcgatcgca
attatcaaaccggatgctgtcatttccaaaaaagtgctggaaatcaaacgtaaaatcaccaaagcgggtttcattatcgaagccgaacat
aaaaccgtgctgacggaagaacaggtggttaatttttattcacgtatcgcggatcagcgcgactttgaagaatttgtttcgtttatgaccag
cggcctgtcttacattctggtcgtgagtcagggttccaaacacaatccgccgagcgaagaaacggaaccgcagaccgatacggaacc
gaacgaacgttctgaagaccagccggaagtggaagcacaagttaccccgggcatgatgaaaaataaacaggatagtctgcaagaat
 
acctggaacgccagcatctggctcaactgtgtgatatcgaagaagacgcggccaacgtggcgaaattcatggatgcctttttcccgga
cttcaagaaaatgaaaagcatgaaactggaaaaaaccctggccctgctgcgtccgaacctgttccacgaacgtaaagatgacgttctg
cgcatcatcaaagatgaagacttcaaaatcctggaacagcgccaagttgtcctgtctgaaaaagaagcacaggctctgtgcaaagaat
acgaaaacgaagattacttcaacaaactgatcgaaaacatgacctcaggtccgtcgctggcactggttctgctgcgtgataatggcctg
cagtattggaaacaactgctgggtccgcgcacggtcgaagaagccattgaatacttcccggaaagcctgtgtgcacagtttgctatgga
ttctctgccggtgaaccaactgtatggcagtgactccctggaaaccgcggaacgtgaaatccagcatttctttctcgagcaccaccacc
accaccactga (SEQ ID NO:48)
(amino acids)

MLVNKKVINLIDEERKIAAGEMARPQYPEIPLVDSDSEVSEESPCESVQEL
YSIAIIKPDAVISKKVLEIKRKITKAGFIIEAEHKTVLTEEQVVNFYSRIADQRDFEEFVS
FMTSGLSYILVVSQGSKHNPPSEETEPQTDTEPNERSEDQPEVEAQVTPGMMKNKQD
SLQEYLERQHLAQLCDIEEDAANVAKFMDAFFPDFKKMKSMKLEKTLALLRPNLFH
ERKDDVLRIIKDEDFKILEQRQVVLSEKEAQALCKEYENEDYFNKLIENMTSGPSLAL
VLLRDNGLQYWKQLLGPRTVEEAIEYFPESLCAQFAMDSLPVNQLYGSDSLETAERE
IQHFFLEHHHHHH* (SEQ ID NO:49)

### NME8 2-3 E coli optimized

(DNA) 
Atgctggaaaaaaccctggccctgctgcgtccgaacctgttccacgaacgtaaagatgacgttctgcgcatcatcaa
agatgaagacttcaaaatcctggaacagcgccaagttgtcctgtctgaaaaagaagcacaggctctgtgcaaagaatacgaaaacga
agattacttcaacaaactgatcgaaaacatgacctcaggtccgtcgctggcactggttctgctgcgtgataatggcctgcagtattggaa
acaactgctgggtccgcgcacggtcgaagaagccattgaatacttcccggaaagcctgtgtgcacagtttgctatggattctctgccgg
tgaaccaactgtatggcagtgactccctggaaaccgcggaacgtgaaatccagcatttctttccgctgcaaagtaccctgggtctgatta
aaccgcacgcgacgtccgaacagcgcgaacaaattctgaaaatcgtcaaagaagccggcttcgatctgacccaggtgaagaaaatg
tttctgaccccggaacaaacggaaaaaatctatccgaaagtcacgggcaaagatttctacaaagacctgctggaaatgctgagtgttgg
tccgtccatggtcatgattctgaccaaatggaatgcggttgcagaatggcgtcgcctgatgggtccgacggatccggaagaagcaaaa
ctgctgagcccggactctattcgcgctcagtttggcatcagcaaactgaaaaacattgttcatggtgcgtccaatgcgtatgaagcgaaa
gaagttgtgaaccgcctgtttgaagacccggaagaaaatctcgagcaccaccaccaccaccactga (SEQ ID NO:50)
(amino acids)

MLEKTLALLRPNLFHERKDDVLRIIKDEDFKILEQRQVVLSEKEAQALCKE
YENEDYFNKLIENMTSGPSLALVLLRDNGLQYWKQLLGPRTVEEAIEYFPESLCAQF
AMDSLPVNQLYGSDSLETAEREIQHFFPLQSTLGLIKPHATSEQREQILKIVKEAGFDL
TQVKKMFLTPEQTEKIYPKVTGKDFYKDLLEMLSVGPSMVMILTKWNAVAEWRRL

MGPTDPEEAKLLSPDSIRAQFGISKLKNIVHGASNAYEAKEVVNRLFEDPEENLEHHH
HHH* (SEQ ID NO:51)

### Structure and Function of NME family proteins

The inventor previously discovered that the growth factor receptor function of a MUC1 cleavage product, MUC1^{∗}, is activated by ligand induced dimerization of its extra cellular domain and that the ligand of MUC1^{∗} was NM23 (Mahanta et al, 2008). The inventor further demonstrated that it was the dimer form of NM23-H1 that binds to the PSMGFR portion of MUC1^{∗} (Figures 21 and 27) and inhibits differentiation and supports the growth of stem and progenitor cells (Hikita et al, 2008; Smagghe et al, 2013). Conversely, NM23-H1 as a hexamer does not promote or maintain pluripotency; rather it induces differentiation. Exemplary experiments are shown in Figures 3-5. Unlike the dimeric form, NM23-H1 hexamers do not bind to the PSMGFR portion of the MUC1 extra cellular domain. Thus, although NME1 hexamers have a biological function in that they induce differentiation, for brevity herein we refer to NME1 hexamers as a "biologically inactive" multimer because it is an NME multimer that does not induce the starting cells to revert to a less mature state or does not inhibit differentiation of immature cells, or does not maintain an immature state of the cell. Similarly, herein we refer to NME1 dimers as a "biologically active" NME multimer, being an NME multimer that induces the starting cells to revert to a less mature state or inhibits differentiation of immature cells, or maintains the immature state of a cell.

In addition, the inventor discovered that, like NME1 (NM23-H1) dimers, NME7 (NM23-H7) monomers (Lacombe et al, 2000) also bind to and dimerize the extra cellular domain of MUC1^{∗} and alone is sufficient to promote or maintain pluripotency in stem or stem-like cells (see Figure 26 and Figure 27). Further, the inventor discovered that NME1 dimers or NME7 monomers induce pluripotency or induce cells to revert to a less mature state.

We have now discovered that several NM23 family members have stem-related functions in that they promote or induce pluripotency while others or other multimerization states of the same NME protein promote or induce differentiation. NME1 (NM23-H1) promotes stem cell growth and inhibits differentiation when it is a dimer only. NME6 is roughly the same molecular weight as NME1 and in sea sponge (*Suberites domuncula*) it is reported to exist as a dimer (Perina et al, 2011, "Characterization of Nme6-like gene/protein from marine sponge Suberites domuncula" Naunyn-Schmiedeberg's Arch Pharmacol, 384:451-460). We expressed NME6 and showed in an ELISA assay that NME6 binds to the PSMGFR peptide derived from the MUC1^{∗} extra cellular domain (Figure 20). In this binding experiment, human NME6 wild type protein was tested along with a variant (S139G) in which a mutation similar to the S120G mutation that made NME1 prefer dimer formation (Chang et al, 1986) and another variant in which that same region that made NME1 prefer dimer formation was mutated (S139A, V142D, V143A) to match the sequence that occurs in sea sponge, as NME6 in sea sponge reportedly exists as a dimer.

Although NME7 (NM23-H7) is a monomeric protein, the inventor has discovered that it functions like a dimer. It contains two NDPK catalytic domains, is approximately twice the molecular weight of an NME1 or NME6 monomer and is expressed and secreted by human embryonic stem (ES) and induced pluripotent stem (iPS) cells, as well as cancer cells (see Figure 1, Figure 2 and Figure 27). NME8 is another NME family member that has some binding affinity for the MUC1 extra cellular domain. NME8 (Miranda-Vizuete et al, 2003) has 3 domains: A, B and C. When NME8 is expressed as only domains A and B, it is monomeric, while B with C forms oligomers. NME8 may bind to and re-cluster MUC1 to turn off stem-like growth.

An ELISA experiment was performed in which NME8, domains A and B ("1" and "2" in Figure 21) or domains B and C ("2" and "3" in Figure 21) were tested for their ability to bind to the PSMGFR peptide from MUC1^{∗} extra cellular domain and compared to the binding of NME1 dimers (NM23 S120G in Figure 21A). As can be seen in the figure, some binding is observed, but it is far less than the binding of NME1 when it is in dimer form. As can be seen in Figure 21B, the most binding is seen when NME8 is an oligomer, which may indicate that like NME1 hexamers, NME8 mutlimers or oligomers induce differentiation by binding to the extra cellular domain of MUC1 and re-clustering the receptors to occlude the binding site of the biologically active NME multimer.

NME family proteins are differentially expressed at different times of cell and tissue development. Whereas we detect NME6, NME7 and NME1 in embryonic stem cells, only NME1 and NME2 are routinely expressed in adult cells or adult stem cells. Because NME1 forms hexamers which induce rather than inhibit stem cell differentiation, it follows that NME7 is expressed earlier in embryogenesis and in naive state stem cells because they cannot form the differentiation-inducing hexamers. In the earliest stages of embryogenesis, growth and inhibition of differentiation would be the default, with the regulatory function of the hexamer being important in later stages when one wants to initiate differentiation when a certain density of stem cells is reached. In support of our findings, Boyer et al (Boyer et al, 2005, "Core Transcriptional Regulatory Circuitry in Human Embryonic Stem Cells", Cell, Vol. 122, 947-956) reported that pluripotency inducing proteins SOX2 and NANOG bind to the promoter of NME7 but not other NME family members, indicating that it is the first NME protein expressed and is consistent with the notion that it can induce or maintain pluripotency but cannot form the hexamers that turn it off. Boyer et al also report that pluripotency inducing proteins SOX2 and OCT4 bind to the promoter of MUC1, the target receptor of NME7 following cleavage to MUC1^{∗}. SOX2 and OCT4 also bind to the promoter of the MUC1 cleavage enzyme MMP-16. The fact that these pluripotency inducing proteins redundantly bind to the promoters of MUC1, its cleavage enzyme and its ligand, NME7, argues that this subset of proteins is critical to pluripotency and that NME7 is the first of the pluripotency proteins expressed in the developing embryo. NME7 is highly expressed by human stem cells if they are cultured in NM23 variants that prefer dimer formation.

Example 1 describes an experiment in which BGO1v human embryonic stem cells are grown in either NM23-S120G, which has been refolded and purified to exist primarily as a dimer, over a coating of anti-MUC1^{∗} monoclonal antibody MN-C3 or cultured in bFGF over mouse fibroblast feeder cells. Western blots of the resultant cells shows that NME7 is highly expressed in stem cells that have been cultured in NM23-S120G dimers (Figure 1, Part I. C - Lane 1) but only weakly expressed in stem cells cultured in bFGF (Lane 2). We have shown that stem cells cultured in NM23 dimers revert to the naive state (Smagghe et al, 2013), which is a less mature state than the primed state of human stem cells cultured in bFGF as are all commercially available stem cell lines. The fact that more NME7 is expressed in naive state stem cells than primed state stem cells is consistent with NME7 being preferentially expressed in very early stages of embryogenesis but not later in embryo or fetal development.

Analysis of adult tissues has shown that NME7 is either not present at all or barely detectable in adult tissues. Example 4 describes an experiment in which human embryonic stem cells were cultured in either NM23-S 120G dimers over a MUC1^{∗} antibody surface or in bFGF over a surface of mouse fibroblast feeder cells, then analyzed by RT-PCR to measure expression levels of the naive genes versus the primed genes. Figure 6 shows that stem cells cultured in NM23 dimers express higher levels of the naive genes and lower levels of the differentiated, primed genes. These results are consistent with the findings shown in Figure 1C. Comparing Lane 1 to Lane 2 shows that NME7 is expressed to a greater degree in the desirable naive stem cells, which are able to differentiate into any cell type in the human body. Therefore, strategies that increase expression of NME7 in a cell, for example via introduction of nucleic acids capable of causing expression of NME7 or methods that add NME7 protein, or NME1 mutants or variants that prefer dimer formation are strategies that maintain pluripotency, maintain the naïve stem cell state in embryonic or induced pluripotent stem cells, and/or induce pluripotency in more mature cell types, including somatic cells, dermablasts and fibroblasts. These strategies may include ectopic expression of one or more of the pluripotency genes Oct4, Sox2, Nanog, Klf4 or c-myc in addition to NME7 or NME1 dimer forming or dimer mimicking variants. These experiments conclude that NME7 is an earlier form of NM23 that is expressed in a more naive stem cell.

We have already shown that NM23 in dimer form induces stem cells to revert to a more pluripotent state often called the naïve state. Our experiments and those of others have shown that culturing stem cells in bFGF or culturing stem cells over a layer of mouse fibroblast feeder cells (MEFs) drives or maintains stem cells in the less pluripotent state called the "primed" state. Referring to Figure 1(C), these primed stem cells express much less NME7, consistent with the idea that NME7 is associated with a more naive and thus truly pluripotent stem cell state. Since the naive state human stem cells are predicted to be better able to differentiate into functional adult cells, the naive stem cells are the desired cells for research as well as for therapeutic use. Thus, strategies that involve inducing expression of NME7 are desired to obtain cells for therapeutic uses. Conversely, strategies that decrease expression of NME7 in cancers would be anti-cancer therapies.

NME7 exists as a single protein but structurally is comprised of two monomers and so functions as a dimer. NME7 contains two NDPK domains, portions of which bind to the MUC1^{∗} growth factor receptor. Example 1 also describes a binding experiment called a pull-down assay. In this experiment, the MUC1^{∗} extra cellular domain peptide was attached to beads which were then incubated with lysates from BGO1v human embryonic stem cells. After wash steps and release from the beads, species captured by interaction with the MUC1^{∗} peptide were separated on an SDS-PAGE gel then probed with an anti-NME7 antibody. Figure 1(F) - Lane 1 shows that NME7 binds to the MUC1^{∗} extra cellular domain. Portions of the double NDPK domains in NME7 bind to MUC1^{∗} growth factor receptor and dimerize it which activates pathways that maintain pluripotency, induce pluripotency and inhibit differentiation of stem and progenitor cells.

NME6 exists as a dimer, and resists formation of higher order multimers. NME6 dimers bind to MUC1^{∗} growth factor receptor and dimerize it which activates pathways that maintain pluripotency, induce pluripotency and inhibit differentiation of stem and progenitor cells. Like NME1 mutants and variants that prefer dimer formation, both NME6 and NME7 are capable of maintaining and inducing pluripotency and inhibiting differentiation of stem and progenitor cells, including iPS cells.

Like NME1 mutants and variants that prefer dimer formation, NME6 and NME7 can be added exogenously to stem cells (embryonic or induced pluripotent) or progenitor cells to induce growth, maintain them in an undifferentiated state or inhibit their differentiation. NME6 and NME7 can be added exogenously to stem or progenitor cells to induce pluripotency. In addition, nucleic acids encoding NME1 mutants and variants that prefer dimer formation, NME6 and/or NME7, or variants thereof, including single chain variants that behave as dimers, can be introduced into cells to induce the cells to revert to a less differentiated state or to maintain cells in a less mature state.

NME1 and NME2 wild type proteins form tetramers, hexamers and higher order multimers. We have discovered that only NME dimers bind to the PSMGFR portion of the MUC1^{∗} growth factor receptor to support stem and progenitor cell growth while inhibiting differentiation. We have also discovered that NME dimers or NME7 monomers cause cells to revert to a less mature state, for example to induce pluripotency in more mature cells including somatic cells, dermablasts, fibroblasts and the like. We also discovered that NME1 wild type proteins that exist primarily as hexamers and higher order multimers, do not bind to PSMGFR portion of the MUC1^{∗} growth factor receptor and induce differentiation of stem and progenitor cells [Smagghe et al, 2013]. NME1 forms multimers, including dimers, tetramers and hexamers. We have shown that the dimer form of NME1 maintains stem cells in an undifferentiated state and also induces pluripotency in more mature cells. However, as stem cell density increases, the amount of NME1 secreted from the stem cells causes an increase in the local concentration, favoring the formation of hexamers that do not support pluripotency and may in fact bind to a different cell surface receptor and actively trigger differentiation. Therefore, it is advantageous for the maintenance or induction of pluripotency to suppress the NME1 isoform to avoid accumulation of the deleterious hexamers. In contrast, NME7 appears to function as the NME1 dimers. Therefore, it is advantageous to increase expression of, or to add exogenously, NME7 for the maintenance or induction of pluripotency. Even more preferred for the maintenance or induction of pluripotency is to suppress NME1 and increase expression of NME7 or to suppress NME1 and increase relative concentration of NME7 or NME1 dimers. These experiments also show that a method for purposely inducing differentiation is to add NME1 hexamers, or to suspend suppression of NME1. Therefore, it would be a great advantage to down-regulate expression of wild type NME1 in stem or progenitor cells *in vitro* or *in vivo* when it is desirable to have the stem or progenitor cells self-replicate without differentiating. Since NME2 also forms hexamers and higher order multimers, it is also desirable to down-regulate NME2 in addition to down regulation of NME1 while maintaining or inducing pluripotency and where inhibition of differentiation is desired. In another embodiment, NME1 or NME1 and NME2 are down regulated while NME1 mutants and variants that prefer dimer formation, such as NME1 S120G or P96S or similar mutations in NME6 or NME7 are added exogenously or nucleic acids encoding them are caused to be expressed in the cells.

In a preferred embodiment, NME family members that can form higher order multimers, greater than dimer, are suppressed. In a more preferred embodiment, expression of NME1 is suppressed in order to promote or maintain cells in a pluripotent state or to induce cells to revert to a less mature state. Suppression of a particular species can be carried out by a number of methods known to those skilled in the art. A target species can be suppressed at the protein level or nucleic acid level: DNA or RNA. The expressed protein can be suppressed by the use of antibodies or small molecules that inhibit or block the activity or binding site of the protein. Alternatively, expression of protein can be inhibited by using anti-sense nucleic acids, anti-sense DNA, anti-sense RNA, inhibitory RNAs, such as RNAi, shRNA, or siRNA. Small molecules may also be used to block or inhibit expression of the targeted protein.

There are several techniques known in the art for introducing nucleic acids into cells to cause expression or repression of a targeted gene. For example, cells can be transfected or transduced with vectors that include sequences that encode the desired or undesirable gene. Vectors can be viruses, DNA or RNA in nature. Other methods involve introducing to cells mRNA that encodes the gene of interest. Genes targeted for repression can be down-regulated using shRNA or siRNA, anti-sense nucleic acids and the like. Some methods for facilitating entry of nucleic acids encoding genes involve the use of detergents so that the encoding nucleic acid is encapsulated in a liposome. Still other methods involve modifying nucleic acids that encode the genes with moieties such as O-methylation or cholesterol, which facilitate entry of the nucleic acid into the cell (See US Patent Application No. US 2010/0173359, filed July 11, 2008, the contents of which are incorporated by reference herein for its disclosure of the moieties). These latter methods are less harsh than detergents so can be used repeatedly to cause a cell to continuously express or repress a targeted gene. For example, Accell^{™} siRNA is specially modified for stability, target specificity, and uptake by cells without a transfection reagent (Dharmacon, Inc. - Layfayette, Colorado). The Accell^{™} system can be used to down-regulate genes such NME1 or NME1 and NME2 that inhibit pluripotency. Modifications of the Accell^{™} system could be used to modify nucleic acids bearing sequences of genes that are beneficial for stem or progenitor cell growth or induction of pluripotency. Many techniques for gene up-regulation or down-regulation are known to those skilled in the art and can be used to down-regulate NME family proteins that are capable of forming higher order multimers, such as hexamers that induce differentiation. For example, to promote pluripotency or reversion to a less mature state, NME1 and/or NME2 are suppressed with or without up-regulation of genes that maintain or induce pluripotency such as NME1 mutants and variants that prefer dimer formation, NME6 and/or NME7, or variants thereof, including single chain variants that behave as dimers.

Whether down-regulating NME1 or NME2 or up-regulating NME1 mutants and variants that prefer dimer formation, NME6 and/or NME7, or variants thereof, including single chain variants that behave as dimers, or adding exogenous protein forms of NME1 mutants and variants that prefer dimer formation, NME6 and/or NME7, or variants thereof, including single chain variants that behave as dimers, these proteins and nucleic acids can be prepared in time release formulations or encapsulated for delivery to cells over time. Using the methods described above, cell culture, especially stem or progenitor cell culture, can be greatly simplified with greatly reduced time and labor. If expression of the differentiation-inducing NME1 and/or NME2 are suppressed, then one merely needs to add sufficient buffer, with or without adding exogenous NME1 mutants and variants that prefer dimer formation, NME6 and/or NME7 to promote pluripotent stem or progenitor cell growth, inhibit differentiation or to induce pluripotency in more mature cells. In a preferred embodiment, NME1 and/or NME2 are suppressed, while NME7 is upregulated or added exogenously to promote, maintain or induce pluripotency or to induce cells to revert to a less mature state.

Suppression of NME7 greatly inhibits stem-like growth. siRNA suppression of NME7 in human stem cells resulted in great inhibition of growth and induction of differentiation in those cells that remained. Quantification of growth was observed and documented photographically. The amount of inhibition of cell growth as well as the change from stem-like morphology to fibroblast-like morphology was similar to that of cells in which the pluripotency gene Oct4 was suppressed. In addition, RT-PCR showed that suppression of NME7 resulted in a marked increase in the expression of FoxA2, which is a marker of the differentiated state. In these experiments, no growth factor was added and cells were characterized every 24 hours until 96 hours after addition of transfection agents. Exemplary images can be seen in Figure 23 and Figure 25.

Conversely, suppression of NME1 had very little effect on stem-like growth or morphology. RT-PCR showed no significant increase in the expression of differentiation marker, FoxA2. The amount of cell growth and the persistence of stem-like morphology was consistent with that of the negative control experiments in which transfection reagents were added but a scrambled siRNA replaced the NME1-targeting siRNA. Exemplary images can be seen in Figure 22 and Figure 25. Suppression of NME6 had a modestly adverse effect on stem-like growth, with some areas of differentiation observed. Areas of differentiation are seen as thickened areas that appear very thick white or as darkened areas of thick cells piled on top of each other. Exemplary images can be seen in Figures 24 and Figure 25 .

These results show that NME1 can be suppressed to eliminate the negative effect of the hexamers that NME1 forms at increased concentration. In fact, when NME1 is suppressed, which is typically only reduced by 50-75% in siRNA knockdown experiments, this likely decreases the local concentration of NME1 and thus decreases the probability of hexamer formation. If NME1 is suppressed but NME7, or NME6 and NME7 are not knocked down, stem cell growth proceeds and is even enhanced such that no additional recombinant growth factor needs to be added. In fact, the temporary suppression of NME1 causes an increase in the concentration of NME7 which promotes pluripotency and does not form the hexamers that can turn off pluripotent growth and induce differentiation.

Therefore, in a preferred embodiment, NME1 but not NME7 is suppressed in pluripotent stem cells, which enables their growth and inhibits differentiation without the addition of exogenously added growth factors, including the addition of recombinant NMEs. NME7 or NME1 in dimer form may be added exogenously, however to increase cell number.

Knockdown of NME1 had very little effect even after 96 hours with no growth factor added into the media. It is well known that if stem cells are cultured in minimal media absent a stem cell growth factor such as bFGF or NME1 in dimer form, the stem cells rapidly differentiate by 48 hours. This did not occur when only NME1 was knocked down, which left NME6 and NME7, suspected of functioning as NME1 dimers would, available to promote pluripotency (See Figure 11, Figure 22 and Figure 25).

Expression of the NME family member, isoform or variant may be transient, stable or controlled as enabled via inducible expression systems so that expression of the NME family member or variant is transient and can be turned on or off in a controllable manner. *Sustained* expression of a biologically active NME family member or variant, especially NME6 or NME7 isoforms or variants, promotes self-renewal, maintains or induces pluripotency while inhibiting differentiation. Conversely, *transient* expression of the biologically active NME family members or variants, especially NME6 or NME7 isoforms or variants maintains or induces pluripotency while inhibiting differentiation for a limited time period, thereafter, cells would differentiate. Alternatively, expression of NME1 wild type can be activated in order to cause cells to enter differentiation as the biologically inactive hexamer form of NME1 is produced. Similarly, expression of NME6 and/or NME7 can be suppressed to induce differentiation. The expression or suppression of NME proteins can be carried out using several techniques known to those skilled in the art, including the use of expression plasmids, linear nucleic acids for expression or suppression, e.g. siRNA, that may be derivatized with moieties such as cholesterol to facilitate entrance into the cells or nucleus.

Cells to which an NME family member, isoform or variant of the invention is added exogenously or which are caused to express the NME family member or variant include but are not limited to somatic cells, dermablasts, fibroblasts, stem cells, pluripotent stem cells, bone marrow cells, peripheral mobilized blood cells, hematopoietic stem cells, progenitor cells, patient cells, cells bearing a genetic defect or alteration, or feeder cells meant to provide neighboring cells with the secreted NME family member or variant. In addition, the cell to which an NME family member, isoform or variant of the invention is added exogenously or which are caused to express the NME family member or variant can be a patient cell. The patient cell may bear a genetic defect or alteration for which it is desired to revert the cell to a less mature state, to correct or replace defective gene, and cause self-replication of the corrected gene bearing cell. The patient cells may be induced to become pluripotent, using NME family members or variants of the invention for treatments for the patient or another patient that requires treatment with stem or progenitor cells. NME family members or variants added exogenously or made to be expressed by patient cells for generating iPS cells, which can then be directed to differentiate into any desirable cell type. Such cells may then be administered to the patient or another patient for therapeutic purposes.

### NME Family Proteins and Treatment of Cancers

NME7 is secreted by cancer cells as well as stem cells. Some cancer cells express mutant NME1 that preferentially forms dimers and resists the formation of hexamers, meaning that in these cases, the cancer cells exhibit stem-like growth due to the dimeric NME1 growth factor, which does not form hexamers, and as a result does not induce differentiation. In other cancers, the stem-like growth factor function of NME7 can overcome the differentiation-inducing effects of the hexamer. Thus, as a treatment for cancers, NME7 is suppressed. Alternatively, NME1 or NME2, which readily form hexamers are overexpressed as a treatment for cancers. In one aspect of the invention, NME7 is suppressed and NME1 and/or NME2 are overexpressed or exogenously introduced to the affected cells to inhibit stem-like growth. In general, any NME family member that resists differentiation-inducing multimerization states, such as the hexamer form, are suppressed to treat cancers. NME family members that induce differentiation, such as high concentrations of NME1 or NME2, are introduced or overexpressed to treat cancers or other maladies characterized by stem-like growth. The invention contemplates practicing these methods *in vitro, ex vivo* and *in vivo.* The invention also contemplates administering agents that suppress or induce expression of NME family members in a patient to either induce stem-like growth, for example at a site of injury, or to inhibit stem-like growth, for example as a treatment for cancers.

We have shown that NME1 in dimer form functions as a pluripotency factor and as a growth factor, stimulating the growth of both stem cells and cancer cells when added to a minimal serum-free media. Here we report that treating cancer cells with NME1 in hexamer form inhibits cancer cell growth and migration, while treatment with the NME1 dimer did not. Exemplary experiments are shown in Figures 28-30.

Different NME multimers may bind to MUC1 at different sites. Different cleavage enzymes may cleave MUC1 such that the binding site for a differentiation-inducing multimer is not present. For example, on cancer cells, or other cells exhibiting stem-like growth, the enzyme that cleaves MUC1 may be different from the cleavage enzyme in stem cells and thus may cleave MUC1 below the binding site for the multimer that turns off stem-like growth. In such cases, it would be advantageous to suppress the MUC1 cleavage enzyme that is not present in primed stem cells. To treat cancer, it may be advantageous to cause expression in cancer cells of the cleavage enzyme that is present in primed stem cells.

Thus, increasing the amount of an NME family member whose multimerization state is the biologically active state or decreasing the relative amount of an NME family member whose multimerization state is the biologically inactive state is a method for generating less mature cells from starting cells or for maintaining cells in a stem-like state, which may be a pluripotent state. Similarly, increasing the amount of an NME family member whose multimerization state is the biologically active state or decreasing the relative amount of an NME family member whose multimerization state is the biologically inactive state is a method of inhibiting differentiation of embryonic stem cells, induced pluripotent stem cells or progenitor cells.

Conversely, for the treatment of cancers, agents that increase the amount of an NME family member whose multimerization state is the biologically inactive state or agents that decrease the relative amount of an NME family member whose multimerization state is the biologically active state are administered to a patient.

Nucleic acids that cause the expression of an NME family member whose multimerization state is the biologically active state, or nucleic acids that suppress expression of an NME family member whose multimerization state is the biologically inactive state, can be introduced to a host cell to promote induction to or maintain it in a less mature or stem-like state. The host cell may also carry a nucleic acid that causes the expression of a gene that is either not expressed in the host cell or is mutated in the host cell. In the case of a defective gene, a nucleic acid that down-regulates the unwanted gene and up-regulates a desired or corrected gene can also be introduced to a host cell. The source of host cells can be a donor or a patient and may be derived from the skin, tooth, bone marrow, blood, placenta, amniotic fluid, a blastocyst, fetus and the like and can be stem cells, iPS cells, progenitor cells or somatic cells. Host cells used with methods of the invention can be administered to a patient. Methods of administering the cells to a patient include by bone marrow transplant, transplant into a specific site, transfusion, injection, or topical treatment. These methods can be used to treat any condition or disease wherein the disease or condition would be alleviated by treatment with stem cells, progenitors, or by correction of a genetic abnormality or defect. Cells subjected to methods of the invention can also be differentiated to progenitors or somatic cells prior to administering to a patient. In cases where differentiation is desired, NME family members whose multimerization state is the biologically active state would be withdrawn to allow differentiation.

Because NME is highly conserved among all species, the methods described herein are not intended to be limited to human NME species nor limited to use with human cells.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. The following examples are offered by way of illustration of the present invention, and not by way of limitation.

### EXAMPLES

**Example 1.** To determine whether or not human stem cells express NME6 or NME7 in addition to NME1 (H1) and NME2 (H2), we performed Western blot analysis on lysates and supernatant from various human stem cell lines. Human embryonic stem cell line BGO1v cells were cultured either in a) NM23-S120G in dimer form only on a cell culture plate coated with anti-MUC1^{∗} monoclonal antibody MN-C3; or b) bFGF at 4 ng/mL on mouse feeder cells (MEFs). After 3 days in culture, the stem cells were harvested and lysed, then analyzed by Western blot using antibodies to probe for the presence of NME1, NME6 and NME7. For comparison, the same analysis was done in parallel on T47D MUC1^{∗}-positive breast cancer cells. As a control, recombinant NM23-H1 wild type (NM23-wt) protein was loaded onto the gel and also probed with antibodies that recognize the 3 different NMEs. Note that the gel is a denaturing gel so that the apparent molecular weight of the NM23-S120G dimer and the wild type hexamer will both appear to be the weight of a monomer. The antibodies used to probe the gel were: for NME1: NM23-H1 (C-20); NME6: NM23-H6 (L-17) and NME7: nm23-H7 (B9) (all purchased from Santa Cruz Biotechnology, Inc).

Figure 1 shows photos of 6 Western blot gels. Part I. A-C shows the Western blots wherein the cell lysate was separated by gel electrophoresis and then probed with antibodies for: A) NME1, B) NME6 and C) NME7. In each panel, Lane 1 corresponds to BGO1v stem cells cultured in NM23-S120G (in dimer form) on a cell culture plate coated with anti-MUC1^{∗} monoclonal antibody MN-C3; Lane 2 corresponds to BGO1v stem cells cultured in bFGF on MEFs; Lane 3 corresponds to T47D breast cancer cells; Lane 4 corresponds to purified recombinant NM23-H1 wild type (NM23-wt).

Figure 1(A) shows that NME1 is present in BGO1v human embryonic stem cells, whether cultured in NM23 in dimer form on an anti-MUC1^{∗} antibody surface (Lane 1) or cultured in bFGF on a surface of mouse feeder cells (MEFs) (Lane 2). NME1 is also present in human breast cancer cells (Lane 3). And the positive control, Lane 4, shows that the antibody used does in fact recognize NME1 purified protein.

Figure 1(B) shows that NME6 is not present in any of the samples tested, using these antibodies.

Figure 1(C) shows that NME7 is strongly expressed in human stem cells if they are cultured in NM23 (dimers) on an anti-MUC1^{∗} surface (Lane 1) but only weakly expressed in stem cells cultured in bFGF on MEF feeder cells (Lane 2). NME7 is also strongly expressed in human breast cancer cells (Lane 3), but is not recognized by the C-20 antibody purportedly specific for the H1 isoform.

Figures 1(D-F) show photos of Western blots of pull-down assays to determine which NMEs bound to the MUC1^{∗} extra cellular domain peptide. Here, a histidine-tagged MUC1* extra cellular domain peptide (GTINVHDVETQFNQYKTEAASRYNLTISDVSVSDVPFPFSAQSGA-HHHHHH) was attached to NTA-Ni agarose beads and then incubated with the same cell lysates as in Part I of Figure 1. After 1 hour incubation at 4 degrees C, beads were centrifuged for 5 minutes at 15000 RPMs. Supernatant was discarded and beads were washed with PBS to remove species bound by non-specific binding. Imidazole was added to release the complex from the beads. After centrifugation, the supernatant was separated by gel electrophoresis and analyzed as in Figure 1, Part I with antibodies against NME1 (D), NME6 (E) and NME7 (F). Figure 1(D) shows that NME1 in stem cells, whether cultured in NM23 (dimers) (Lane 1) or in bFGF (Lane 2), binds to MUC1^{∗} extra cellular domain peptide, as the inventor has previously shown. Lane 3 shows that NME1 in breast cancer cell lysates also binds to MUC1^{∗} extra cellular domain peptide and Lane 4 shows that the C-20 NME1 specific antibody binds to the purified recombinant wild type NME1. Referring to Figure 1(E), this gel shows that NME6, which in Part I of Figure 1 was shown not to be in these cell lysates, was also not pulled down by the MUC1^{∗} peptide. Figure 1(F) importantly shows that NME7 binds to the MUC1^{∗} extra cellular domain peptide. NME7 from stem cells cultured in NM23 dimers and over a MUC1^{∗} antibody surface expressed greater amounts of NME7 than stem cells cultured in bFGF over MEFs. Consistent with Part I of Figure 1, NME7 was shown to bind to MUC1^{∗} peptide and was pulled down in the assay by that interaction (Lane 1). However, NME7 does not appear in Lane 2, which is likely due to the reduced expression in cells cultured in bFGF. Lane 3 shows that NME7 expressed in breast cancer cells binds to MUC1^{∗} and Lane 4 shows no protein because the NME7 antibody does not recognize the NME1 isotype. NME7 likely binds to two MUC1^{∗} peptides to dimerize MUC1^{∗} receptors on cells, thus stimulating pluripotency, growth and inhibition of differentiation.

**Example 2.** Western blot analysis of human stem cell lines BGO1v and HES-3 cells shows that an NME antibody, purportedly specific for NME7 recognized NME7 and another species having an apparent molecular weight of ~22-25kDa (Figure 2).

**Example 3.** The inventor previously showed that NM23 in dimer form or in a form that dimerized MUC1^{∗} receptor on cells promoted pluripotent stem cell growth, inhibited differentiation and induced pluripotency in more mature cells including somatic cells. Binding experiments showed that NM23 in dimer form bound to the MUC1^{∗} receptor on cells and to the free MUC1^{∗} extra cellular domain peptide. However, experiments using the hexamer form of either the wild type protein or the hexamer form of the S120G mutant caused stem cells to differentiate. Cellular localization experiments showed that the hexamer could bind to cells and was translocated to the nucleus where it presumably bound to elements that induce differentiation. Therefore, it is clear that expression of NME1 (NM23-H1) that forms hexamers at very low concentration as well as other NME family members that form hexamers and higher order multimers are detrimental to the process of culturing stem cells *in vitro,* inducing pluripotency in more mature cells such as somatic cells and detrimental to the process of seeding a patient with a stem or progenitor cell wherein expansion to reconstitute a stem cell population or to overtake an endogenous stem cell population are desired.

To show the deleterious effect of NME1 wild type, we cultured human stem cells, BGO1vs, in either all NME1-S120G dimers (Figure 3), all NME-wt which are hexamers (Figure 4), or mixtures of the two wherein the NME1-S120G dimer was at 20% and the NME-wt was at 80% and the total NME concentration ranged from 8nM - 104nM (Figure 5). Figures 3-5 are photographs of the resultant stem cells on Day 4 post plating. Areas of darkened and thickened cells indicate that the stem cells have differentiated in contrast to the pluripotent stem cells which are a single layer of clear, small cells with large nucleus to cytoplasm ratio. The images clearly show that NM23 dimers (NME1-S120G dimers) fully support pluripotent stem cell growth (Figure 3) while the hexameric form makes cells differentiate (Figure 4). Figure 5 shows that the presence of a significant amount of NME1 wild type, which is hexameric, causes the stem cells to differentiate despite the presence of the dimer. Thus, the wild type NME1 endogenous to human stem cells and secreted by them inhibits the growth and maintenance of stem and progenitor cells and inhibits the induction of pluripotency in more mature cells such as somatic cells.

**Example 4.** RT-PCR of naive versus primed markers for human stem cells cultured in NM23 dimers compared to cells cultured in bFGF. Human embryonic H9 stem cells were plated onto plasticware coated with an anti-MUC1^{∗} monoclonal antibody MN-C3 then cultured in NM23-S120G, purified to dimer population only, for 37 passages. Samples of the growing cells were withdrawn at passages 8, 12, 14, 21, 26 and 37. For comparison, cells from the same starting stock were cultured in bFGF over a layer of mouse feeder cells (MEFs). Cells were analyzed using standard quantitative PCR techniques. Expression levels of OCT4 and NANOG (pluripotency genes), KLF4 and KLF2 (naive genes) and LHX2, OTX2, XIST and FOXa2 (primed genes) were measured relative to GAPDH and normalized to their expression in cells cultured in bFGF over MEFs. As can be seen in Figure 6, stem cells cultured in NM23 dimers (NME1-S120G dimers) express high levels of the naive genes and lower levels of the primed genes, indicating the cells are in the more primitive, naïve state than stem cells culture in bFGF. Together with the results of Example 1 shown in Figure 1, Part I. C), NME7 is characteristic of the naive state, not the primed state, and is better able to support pluripotent stem cell growth. These data provide rationale for adding exogenous NME7 or nucleic acids that induce its expression for the maintenance of pluripotency in cells, inhibition of differentiation, or the induction of pluripotency in cells. Such introduction of NME7 can be accompanied by repression of the differentiation-inducing NME1.

**Example 5.** Suppression of NME family members.

To demonstrate the deleterious effect of some NMEs and the beneficial effects of other NMEs on the maintenance or induction of pluripotency, we performed knockdown experiments in which siRNA was used to knock down one or more of NME1, NME6 and NME7 in pluripotent stem cells. siRNA was added to established ES and iPS cells that are commercially available. Cells were cultured in serum-free minimal stem cell growth media and pluripotent stem cells were plated over a layer of an antibody to a stem cell surface receptor, MUC1*, to avoid introduction of random growth factors that are present in feeder cells and Matrigel.

NME1, NME6 and NME7 were all knocked down together to assess the effect of knocking down NM23s without recognizing that some promote differentiation and others inhibit it. As can be seen in Figure 7A, suppressing expression of NME1, NME6 and NME7 results in poor cell growth and induces differentiation as can be seen by thickened areas of cell growth, in which cells have smaller nuclei and take on fibroblast-like morphology. Knocking down NM23, without distinguishing among which isoforms are deleterious and which are good for the maintenance of pluripotency, causes differentiation within 48 hours. In the control experiment, equal amounts of a scrambled siRNA were added but no induction of differentiation is observed (Figure 7B).

In single knockdown experiments, suppression of NME1 had little or no effect and there was no significant differentiation within the first 48 hours (Figure 8A). Suppression of NME6 alone, has a modest effect, but at 48 hours does not appear to be significantly different from the NME1 knockdown (Figure 8B). In stark contrast, down regulation of NME7 has a dramatic and negative effect on stem cell growth and induces differentiation (Figure 8C). In the triple and double knockdown experiments (Figures 8D-F), suppressing NME1 and NME7 together induced differentiation and suppressed cell growth (Figure 8F), but comparison to Figure 8A in which NME1 alone is suppressed implies that the effect is due to 2-times as much siRNA added rather than a synergistic effect between NME1 and NME7.

We next sought to determine if the addition of recombinant NME1 dimers could rescue differentiation induced by suppression of NME7. siRNA specific for either NME1 or NME7 (Figure 9A, B respectively) was added to pluripotent stem cells in culture as described above. In identical wells the siRNA was added but in addition, recombinant NME1 dimer was added. As can be seen in Figure 9D, addition of recombinant NME1 dimer prevented that differentiation that occurs when NME7 is suppressed. Addition of recombinant NME1 dimers increases the growth of pluripotent cells wherein NME1 is suppressed, but there is no significant differentiation in either case.

Inhibition of cell growth and differentiation induced by knock down of all three NM23 isoforms (Figure 10A) is rescued in part by the addition of recombinant NME1 purified as stable dimers (Figure 10B).

siRNAs specific for NME1, NME6 and NME7 were purchased from Santa Cruz Laboratories, Santa Cruz, CA. The procedure used was the Stemgen^{t™} siRNA transfection kit. The method uses lipids to form siRNA micelles, which will permeate the cell membrane and then act on the targeted RNA to downregulate it.

As negative controls, a scrambled sequence siRNA was used and mock transfection was done in which reagents were added but no siRNA was added. As a positive control, OCT4 siRNA was added. To assess the effects of multiple knockdowns in the same cell, scrambled siRNA was added at 3-times the concentration of a single knockdown. OCT4 is a pluripotency gene so suppressing its expression should result in stem cell differentiation.

**Example 6.** The experiment of Example 5 was continued to 96 hours. Evaluation of cell morphology confirmed the 48 hour observations, but with additional caveats. After 96 hours, the NME variant knockdown experiment was extended to analyze the expression of pluripotency genes as well as to analyze the effect of suppressing one NME isoform on the other NME isoforms. Stem cells from each experimental condition were separately pelleted and analyzed by RT-PCR. Expression levels of pluripotency genes Nanog, Oct4, and Klf4, along with differentiation gene Foxa2 and NME isoforms NME1, NME6 and NME7 were measured. Expression levels were normalized to a control mock transfection of siRNA. siRNA typically suppresses the targeted gene by 50-75%. As a positive control, siRNA specific for Oct4 was used. Stem cells transfected with Oct4 siRNA differentiated as expected even if cultured in normal stem cell media and growth factor.

Results: Referring to Figure 14, it can be seen that in the differentiation control in which pluripotency gene Oct4 is knocked down, differentiation gene Foxa2 is elevated and expression of all three NME genes is suppressed. The same differentiation gene Foxa2 is also elevated when NME1, NME6 or NME7 is knocked down. However, it can also be seen that when one NME gene is knocked down, there appears to be an increase in one or both of the other NME genes. When NME1 is knocked down, NME6 is increased. When NME6 is knocked down NME 1 and NME7 are increased. When NME7 is knocked down NME1 is increased.

When the recombinant dimer form of NME1 is added into the media there is a rescue effect. Figure 15 shows that when NME1 is knocked down there is an increase in the expression of differentiation gene Foxa2, although the increase is minimal compared to its expression level in the differentiation control. This is consistent with the photos of Figure 11A and Figure 12A which show no visible signs of differentiation in stem cells in which NME1 has been knocked down. When the recombinant NME1 dimer is added into the media, Figure 15 shows that there is a significant decrease in the differentiation gene Foxa2 and an increase in the expression of NME1 and NME7. The photos of Figure 11B and Figure 12B are consistent with this result in that they show that there is an increase in the number of undifferentiated stem cells, but no significant differentiation of the cells whether the recombinant NME1 dimers are added in or not. This result indicates that NME1 can be down regulated or knocked out in pluripotent stem cells without a significant adverse effect, because NME6 or NME7, which function as dimers are still present.

Referring now to Figure 16, siRNA suppression of NME6 results in only a modest increase in the differentiation marker Foxa2. However, when recombinant NME1 dimers are added into the media, there is a large increase in expression of the differentiation gene Foxa2, which is however accompanied by a large increase in expression of the pluripotency gene Klf4 and stimulation of expression of NME6 itself. Inspection of the corresponding cells showed that when NME6 was suppressed, there was a decrease in cell growth and a very modest increase in differentiation. The results suggest that NME6 is present in pluripotent stem cells, but its role may not be critical in that NME6 alone can be knocked down without a deleterious effect. However, adding in recombinant NME1 dimers may not improve growth or inhibition of differentiation.

Knock down of NME7 had the largest and most negative effect on cell growth and cells differentiated rapidly (see Figure 11B and Figure 12B). The addition of recombinant NME1 dimers had a modest rescue effect, wherein the cells appear to be less differentiated (Figure 11D and Figure 12D). Figure 17 shows that the NME7 knockdown has increased levels of differentiation gene Foxa2 which are reduced when NME1 dimers are added into the media. Addition of recombinant NME1 dimers also reduced the level of NME1 expression to compensate for the absence of NME7.

Figure 13 shows that all three NMEs cannot be knocked down without having a disastrous effect on cell growth and differentiation. The triple knockdown gave rise to very little cells that were alive after 96 hours and those cells were entirely differentiated (Figure 13A). Knock down of NME1 and NME7 had an identical disastrous effect (Figure 13B), implying that the role of NME6 is not as important as that of NME1 and NME7. The addition of recombinant NME1 in dimer form improved cell growth but only to a limited degree (Figure 13C, D). Figure 18 shows that the triple knockdown causes an increase in the differentiation gene Foxa2 and greatly reduced expression of all the NMEs. When recombinant NME1 dimers are added to the media, the level of Foxa2 is reduced but the levels of the pluripotency genes and the NME genes is comparable to the levels expressed in the differentiation control. Figure 19 shows that there is a very similar pattern of gene expression when NME1 and NME7 are knocked down together.

**Example 7.** In another NME knockdown experiment performed as described above, human embryonic stem cells (BGO1V) were plated over a surface of an anti-MUC1^{∗} antibody (MN-C3). Specific NME proteins were knocked down using siRNA and images as well as RT-PCR quantified the results at 96 hours post transfection of the siRNA. No growth factor or serum was added to the cells after siRNA transfection. A minimal stem cell media was changed every 48 hours. Figures 22A,B show photographs of the stem cells 96 hours after NME1 was suppressed and in the absence of any growth factors or serum added. Note that the cell number and cell morphology appear stem-like and indistinguishable from the negative control experiment shown in Figure 22C,D in which transfection reagents were added but a nonsense siRNA was added. These results show that suppressing NME1 has no deleterious effect on stem-like growth. By contrast, Figure 23A,B shows that suppression of NME7 inhibited stem-like growth and inhibited stem cell growth in general. The one remaining cluster of cells is differentiating as can be seen by the thicker, whiter clustering of cells at the center of Figure 23B. By comparison to the positive control, suppressing pluripotency gene Oct4 (Figures 23C,D), it can be seen that suppression of NME7 is comparable to suppression of the key pluripotency gene Oct 4. Suppression of NME6, shown in Figures 24A,B inhibited stem-like growth to a modest extent as areas of differentiation can be seen in the view shown in Figure 24B. However, NME6 suppression looks similar to the negative control cells wherein scrambled siRNA was added (Figures 24C,D).

RT-PCR of the resultant NME suppression experiment described immediately above, shows that only suppression of NME7 resulted in a significant increase in the expression of Foxa2, which is a marker of differentiation.

**Example 8.** Recombinant human NME7 promotes and maintains pluripotent stem cell growth

Testing recombinant NME7 for ability to maintain pluripotency and inhibit differentiation. A soluble variant of NME7, NME7-AB, was generated and purified as previously described (PCT/US12/60684, filed October 17, 2012, the contents of which are incorporated by reference in their entirety). Human stem cells (iPS cat# SC101a-1, System Biosciences) were grown per the manufacturer's directions in 4ng/ml bFGF over a layer of mouse fibroblast feeder cells for four passages. These source stem cells were then plated into 6-well cell culture plates (Vita^{™}, Thermo Fisher) that had been coated with 12.5 ug/well of a monoclonal anti-MUC1^{∗} antibody, MN-C3. Cells were plated at a density of 300,000 cells per well. The base media was Minimal Stem Cell Media consisting of: 400 ml DME/F12/GlutaMAX I (Invitrogen# 10565-018), 100 ml Knockout Serum Replacement (KO-SR, Invitrogen# 10828-028), 5 ml 100x MEM Non-essential Amino Acid Solution (Invitrogen# 11140-050) and 0.9 ml (0.1mM) β-mercaptoethanol (55mM stock, Invitrogen# 21985-023). The base media can be any media. In a preferred embodiment, the base media is free of other growth factors and cytokines. To the base media was added either 8nM of NME7-AB or 8nM NM23-H1 refolded and purified as stable dimers. Media was changed every 48 hours and due to accelerated growth had to be harvested and passaged at Day 3 post-plating. Figure 26 shows that culturing human stem cells in NM23-H1 dimers or in NME7 monomers results in pluripotent stem cell growth. NME7 and NM23-H1 (NME1) dimers both grew pluripotently and had no differentiation even when 100% confluent. As can be seen in the photos, NME7 cells grew faster than the cells grown in NM23-H1 dimers. Cell counts at the first harvest verified that culture in NME7 produced 1.4-times more cells than culture in NM23-H1 dimers.

**Example 9.** ELISA assay showing NME7-AB simultaneously binds to two MUC1 * extra cellular domain peptides

The PSMGFR peptide bearing a C-terminal Cysteine (PSMGFR-Cys) was covalently coupled to BSA using Imject Maleimide activated BSA kit (Thermo Fisher). PSMGFR-Cys coupled BSA was diluted to 10ug/mL in 0.1M carbonate/bicarbonate buffer pH 9.6 and 50uL was added to each well of a 96 well plate. After overnight incubation at 4°C, the plate was washed twice with PBS-T and a 3% BSA solution was added to block remaining binding site on the well. After 1h at room temperature the plate was washed twice with PBS-T and NME7, diluted in PBS-T + 1% BSA, was added at different concentrations. After 1h at room temperature the plate was washed 3x with PBS-T and anti-NM23-H7 (B-9, Santa Cruz Biotechnology), diluted in PBS-T + 1% BSA, was added at 1/500 dilution. After 1h at room temperature the plate was washed 3x with PBS-T and goat anti mouse-HRP, diluted in PBS-T + 1% BSA, was added at 1/3333 dilution. After 1h at room temperature the plate was washed 3x with PBS-T and binding of NME7 was measured at 415nm using a ABTS solution (Pierce).

ELISA MUC1^{∗} dimerization: The protocol for NME7 binding was used and NME7 was used at 11.6ug/mL.

After 1h at room temperature the plate was washed 3x with PBS-T and HisTagged PSMGFR peptide (PSMGFR-His) or biotinylated PSMGFR peptide (PSMGFR-biotin), diluted in PBS-T + 1% BSA, was added at different concentration. After 1h at room temperature the plate was washed 3x with PBS-T and anti Histag-HRP (Abcam) or streptavidin-HRP (Pierce), diluted in PBS-T + 1% BSA, was added at a concentration of 1/5000. After 1h at room temperature the plate was washed 3x with PBS-T and binding of PSMGFR peptide to NME7 already bound to another PSMGFR peptide (which could not signal by anti-His antibody or by streptavidin) coupled BSA was measured at 415nm using a ABTS solution (Pierce).

**Example 10.** NME1 in hexamer form inhibits cancer cell growth and migration.

T47D breast cancer cells secrete both NME1 and NME7. T47D MUC1^{∗}-positive breast cancer cells were cultured in varying amounts of NME1 that had been purified as a stable population of essentially all hexamers in order to determine whether or not hexameric NME1 inhibited cancer cell growth and stem-like growth in the same manner stem cell growth is inhibited. T47D cells were grown in 10% FBS RPMI and collected with trypsin. Cells were spun down and counted with a hemacytometer. 6,000 cells per well were seeded into 96 well plates. 24 hours later the media was changed to 1% FBS RPMI plus the varying concentrations of hexamer. Media was again changed 48 hours later. Cell viability was measured at 96 hrs using the Vialight Plus (Lonza, Rockland ME) cell assay which measures the amount of ATP present in the cells. Data is graphed as the percent cell growth relative to the media only control. As another control, a small molecule (MN547) that we identified by virtue of its ability to inhibit cancer cell growth was included as a positive control. Figure 28 shows that NME1 in hexamer form inhibits the proliferation of breast cancer cells in a concentration dependent manner.

**Example 11.** NME1 in hexamer form inhibits cancer cell migration; NME1 dimers do not.

Cancer cell migration is often regarded as one of the methods by which cancer cells invade other tissues or implant at various locations to start occult metastases. One method of testing for inhibitors of cancer cell migration is a "scratch test." In these assays, cancer cells are plated and allowed to proliferate to near confluency. A pipette tip or other instrument is used to scrape off a section of cells. Dislodged cells are washed away, leaving a background of growing cancer cells with an "slash" or "cross" shaped region in each well that is devoid of cells. NME1 purified as a pure population of either hexamers or dimers was added and allowed to grow for an additional 16 or 24 hours.

DU145 MUC1^{∗}-positive prostate cancer cells were grown in 10% FBS RPMI and collected with trypsin. Cells were spun down and counted with a hemacytometer. Approximately 1 × 10⁶ cells per well were plated into 6 well plates in 10% FBS RPMI. The following day either a cross mark (NME1 hexamers) or a slash mark (NME1 dimers) was made using a sterile 1000 uL pipette tip to remove cells in a defined region. Dislodged cells were gently washed away. The media was changed to 10% FBS plus the varying concentrations of hexamer or dimer. Photographs were taken at the time of the scratch (t = 0) and again 16 hours later using the cross of the lines as the center of the picture. For time zero and for time 16 hours, the area of the scratch was quantitated using ImageJ software. Data is expressed as the percent of cellular invasion into the scratch relative to its own zero hour photograph.

Figure 29A shows the graph of the Image J quantification of the percent invasion for cancer cells treated with NME1 hexamers at the concentrations shown. Figures 29B-E show photographs at time zero (B,C) and at 16 hours (D,E). By comparison to the control (D), it is clear that NME1 hexamer (E) inhibits cancer cell invasion.

Figure 30A shows the graph of the Image J quantification of the percent invasion for cancer cells treated with NME1 dimers at the concentrations shown. Figures 30B-K show photographs at time zero (B-F) and at 16 hours (G-K). By comparison to the control (G), it is clear that NME1 dimers (H,I) do not inhibit cancer cell invasion. Recall that NME1 dimers bind to and dimerize the extra cellular domain of the MUC1^{∗} growth factor receptor on cancer cells to stimulate growth. As we have shown with stem cells, concentrations of the NME1 dimers of ~4-16nM are sufficient such that one NME1 dimer binds to two (2) MUC1^{∗} receptors. At higher concentrations (J,K), there would be one dimer bound to each MUC1^{∗} receptor rather than dimerizing the two receptors. Thus at very high NME1 dimer concentration, there is an inhibition of invasion.

### REFERENCES

Boissan, M., et al. (2009). "The mammalian Nm23/NDPK family: from metastasis control to cilia movement." Mol Cell Biochem 329(1-2): 51-62.
Okabe-Kado, J., et al. (1985). "Characterization of a differentiation-inhibitory activity from nondifferentiating mouse myeloid leukemia cells." Cancer Res 45(10): 4848-52.
Okabe-Kado, J., et al. (1992). "Identity of a differentiation inhibiting factor for mouse myeloid leukemia cells with NM23/nucleoside diphosphate kinase." Biochem Biophys Res Commun 182(3): 987-94.
Rosengard, A. M., et al. (1989). "Reduced Nm23/Awd protein in tumour metastasis and aberrant Drosophila development." Nature 342(6246): 177-80.
Dearolf, C. R., et al. (1988). "Developmental consequences of awdb3, a cell-autonomous lethal mutation of Drosophila induced by hybrid dysgenesis." Dev Biol 129(1): 159-68.
Timmons, L., et al. (1993). "The expression of the Drosophila awd gene during normal development and in neoplastic brain tumors caused by lgl mutations." Dev Biol 158(2): 364-79.
Yamashiro, S., et al. (1994). "Alteration of nm23 gene expression during the induced differentiation of human leukemia cell lines." Oncogene 9(9): 2461-8.
Lombardi, D., et al. (1995). "The association of the Nm23-M1 protein and beta-tubulin correlates with cell differentiation." Exp Cell Res 217(2): 267-71.
Gervasi, F., et al. (1996). "nm23 influences proliferation and differentiation of PC12 cells in response to nerve growth factor." Cell Growth Differ 7(12): 1689-95.
Amendola, R., et al. (1997). "DR-nm23 gene expression in neuroblastoma cells: relationship to integrin expression, adhesion characteristics, and differentiation." J Natl Cancer Inst 89(17): 1300-10.
Munoz-Dorado, J., et al. (1990). "Nucleoside diphosphate kinase from Myxococcus xanthus. I. Cloning and sequencing of the gene." J Biol Chem 265(5): 2702-6.
Okabe-Kado, J., et al. (1995). "Inhibitory action of nm23 proteins on induction of erythroid differentiation of human leukemia cells." Biochim Biophys Acta 1267(2-3): 101-6.
Venturelli, D., et al. (1995). "Overexpression of DR-nm23, a protein encoded by a member of the nm23 gene family, inhibits granulocyte differentiation and induces apoptosis in 32Dc13 myeloid cells." Proc Natl Acad Sci U S A 92(16): 7435-9.
Willems, R., et al. (1998). "Decrease in nucleoside diphosphate kinase (NDPK/nm23) expression during hematopoietic maturation." J Biol Chem 273(22): 13663-8.
Willems, R., et al. (2002). "Extracellular nucleoside diphosphate kinase NM23/NDPK modulates normal hematopoietic differentiation." Exp Hematol 30(7): 640-8.
Lombardi, D., et al. (2000). "nm23: unraveling its biological function in cell differentiation." J Cell Physiol 182(2): 144-9.
Ptashne, M. (2004). A Genetic Switch, Phage Lambda Revisited. Cold Spring Harbor, Cold Spring Harbor Laboratory Press.
Ieda, M., et al. (2010). "Direct reprogramming of fibroblasts into functional cardiomyocytes by defined factors." Cell. 142(3): 375-86.
Efe, J. A., et al. (2011). "Conversion of mouse fibroblasts into cardiomyocytes using a direct reprogramming strategy." Nat Cell Biol. 13(3): 215-22.
Nichols, J. and A. Smith (2009). "Naive and primed pluripotent states." Cell Stem Cell 4(6): 487-92.
Mahanta, S., et al. (2008). "A minimal fragment of MUC1 mediates growth of cancer cells." PLoS One. 3(4): e2054.
Hikita, S. T., et al. (2008). "MUC1∗ mediates the growth of human pluripotent stem cells." PLoS One. 3(10): e3312.
Smagghe, B. J., et al. (2013). "MUC1* ligand, NM23-H1, is a novel growth factor that maintains human stem cells in a more naive state." PLoS One. 8(3): e58601.
Lacombe, M. L., et al. (2000). "The human Nm23/nucleoside diphosphate kinases." J Bioenerg Biomembr 32(3): 247-58.
Perina, D., et al. "Characterization of Nme6-like gene/protein from marine sponge Suberites domuncula." Naunyn Schmiedebergs Arch Pharmacol 384(4-5): 451-60.
Chang, C. L., et al. (1996). "A nucleoside diphosphate kinase A (nm23-H1) serine 120-->glycine substitution in advanced stage neuroblastoma affects enzyme stability and alters protein-protein interaction." Oncogene 12(3): 659-67.
Miranda-Vizuete, A., et al. (2003). "Cloning and developmental analysis of murid spermatid-specific thioredoxin-2 (SPTRX-2), a novel sperm fibrous sheath protein and autoantigen." J Biol Chem 278(45): 44874-85.
Boyer, L. A., et al. (2005). "Core transcriptional regulatory circuitry in human embryonic stem cells." Cell 122(6): 947-56.

All of the references cited herein are incorporated by reference in their entirety.

Those skilled in the art will recognize, or be able to ascertain using no more than routine experimentation, many equivalents to the specific embodiments of the invention specifically described herein. Such equivalents are intended to be encompassed in the scope of the claims.

The following paragraphs define embodiments of the invention.
1. A method for generating less mature cells from starting cells comprising inducing the starting cells to revert to a less mature state comprising increasing the amount of an NME family member whose multimerization state is the biologically active state or decreasing the relative amount of an NME family member whose multimerization state is the biologically inactive state.
2. A method of inhibiting differentiation of embryonic stem cell, induced pluripotent stem cell or progenitor cell comprising increasing the amount of an NME family member whose multimerization state is the biologically active state or decreasing the relative amount of an NME family member whose multimerization state is the biologically inactive state.
3. A method of maintaining or inducing pluripotency in cells comprising decreasing the relative amount of an NME family member whose multimerization state is the biologically inactive state.
4. A method of inhibiting differentiation in embryonic stem cell, induced pluripotent stem cell, or progenitor cell, comprising decreasing the relative amount of an NME family member whose multimerization state is the biologically inactive state.
5. A method according to any of the preceding paragraphs, wherein the NME family member's multimerization state in the biologically inactive state is a hexamer or higher order multimer.
6. A method according to any of the preceding paragraphs, wherein the NME family member's multimerization state in the biologically active state is a dimer or a monomer that contains two units able to bind to the same target receptor.
7. The method according to paragraph 5, wherein the NME family member is NME1 (NM23-H1).
8. The method according to paragraph 5, wherein the NME family member is NME2 (NM23-H2).
9. The method according to paragraph 6, wherein the NME family member is a mutant or variant of NME1 that favors dimerization or has two binding domains for its cognate ligand.
10. The method according to paragraph 6, wherein the NME family member is NME7.
11. The method according to paragraph 6, wherein the NME family member is NME6.
12. The method according to any of the preceding paragraphs, wherein the relative amount of the NME family member whose multimerization state is the biologically inactive state is decreased by adding an NME family member whose multimerization state is the biologically active state.
13. The method according to paragraph 12, wherein the NME family member whose multimerization state is the biologically active state is increased by introducing a nucleic acid or small molecule that causes it to be expressed.
14. The method according to any of the preceding paragraphs wherein the relative amount of the NME family member whose multimerization state is the biologically inactive state is decreased by introducing nucleic acids or small molecules that down-regulate its expression.
15. The method according to any of the preceding paragraphs, wherein the NME family member whose multimerization state is the biologically inactive state is decreased and the NME family member whose multimerization state is the biologically active state is increased by simultaneously introducing a first nucleic acid that down-regulates a first NME that forms the inactive state and a second nucleic acid that up-regulates the NME that forms the active state.
16. The method according to paragraphs 14 or paragraph 15, in which the nucleic acid that down-regulates is an anti-sense DNA, an inhibitory RNA, siRNA or shRNA and the nucleic acid that up-regulates is an encoding DNA, RNA, mRNA, or plasmid.
17. The method according to paragraph 16, in which the nucleic acid is modified to facilitate entry into the cell.
18. The method according to paragraph 14 to 17, wherein the NME family member that is down regulated is NME1, NME2 or NME1 and NME2.
19. The method according to paragraph 14 to 17, wherein the NME family member that is up-regulated is a mutant or variant of NME1, NME2 that prefers dimerization, NME6 or NME7.
20. A nucleic acid that causes expression of NME1 mutant or variant that prefers dimer formation or has two binding domains of the same ligand; and/or
   a. NME7; and/or
   b. a nucleic acid that causes expression of NME7; and/or
   c. NME6; and/or
   d. a nucleic acid that causes expression of NME6; and/or
   e. A nucleic acid that down-regulates NME1 or NME1 and NME2.
21. A media for culture of stem or progenitor cells wherein the media contains:
   a. NME1 mutant or variant that prefers dimer formation or has two binding domains of the same ligand; and/or
   b. a nucleic acid that causes expression of NME1 mutant or variant that prefers dimer formation or has two binding domains of the same ligand; and/or
   c. NME7; and/or
   d. a nucleic acid that causes expression of NME7; and/or
   e. NME6; and/or
   f. a nucleic acid that causes expression of NME6; and/or
   g. a nucleic acid that down-regulates NME1 or NME1 and NME2.
22. A media for inducing pluripotency or for inducing cells to revert to a less mature state wherein the media contains:
   a. NME1 mutant or variant that prefers dimer formation or has two binding domains of the same ligand; and/or
   b. a nucleic acid that causes expression of NME1 mutant or variant that prefers dimer formation or has two binding domains of the same ligand; and/or
   c. NME7; and/or
   d. a nucleic acid that causes expression of NME7; and/or
   e. NME6; and/or
   f. a nucleic acid that causes expression of NME6; and/or
   g. contains a nucleic acid that down-regulates NME1 or NME1 and NME2.
23. A media as in paragraph 22 wherein the media also contains nucleic acids that encode some or all of the pluripotency-inducing genes or small molecules including OCT4, SOX2, NANOG, KLF4, and c-Myc.
24. A host cell that carries a synthetic nucleic acid that causes decreased expression of an NME family member whose multimerization state is the biologically inactive state.
25. A host cell as in paragraph 24, wherein the NME family member is NME1
26. A host cell as in paragraph 24, wherein the NME family member is NME1 and NME2.
27. A host cell that carries a synthetic nucleic acid that causes increased expression of an NME family member whose multimerization state is the biologically active state.
28. A host cell as in paragraph 27, wherein the NME family member is an NME1 mutant or variant that prefers dimer formation or has two binding domains of the same ligand
29. A host cell as in paragraph 27, wherein the NME family member is NME7.
30. A host cell as in paragraph 27, wherein the NME family member is NME6.
31. A host cell that carries a synthetic nucleic acid that causes decreased expression of an NME family member whose multimerization state is the biologically inactive state and a synthetic nucleic acid that causes increased expression of an NME family member whose multimerization state is the biologically active state.
32. A host cell as in paragraph 31, wherein the NME family member whose expression will be decreased is NME1 or NME1 and NME2 and the NME family member whose expression will be increased is an NME1 mutant or variant that prefers dimer formation or has two binding domains of the same ligand, and/or NME6, and/or NME7.
33. A host cell as in any of the paragraphs 24 to 32 above, wherein the cell also carries a nucleic acid that causes the expression of a gene that is either not expressed in the host cell or is mutated in the host cell.
34. A host cell as in paragraph 33, that also carries a nucleic acid to down-regulate an unwanted gene and up-regulates a desired or corrected gene.
35. A host cell as in any of paragraphs 24 to 34 above, wherein the host cell is embryonic stem cell, induced pluripotent stem cell, progenitor cell, or somatic cell.
36. A method of treating a patient wherein the cells of any of the paragraphs 23 to 35 above are administered to the patient.
37. The method according to paragraph 36, wherein the cells are administered by bone marrow transplant, transplant into a specific site, transfusion, injection, or topical treatment.
38. The method according to paragraph 36, wherein the treatment is for any disease or condition that would be alleviated by treatment with stem cells, progenitors, or by correction of a genetic abnormality or defect.
39. The method according to paragraph 36, wherein the cells are differentiated prior to administration to patient and wherein NME1, NME6 or NME7 are withdrawn during differentiation process.
40. A method of treating cancer in a patient comprising administering an agent to the patient such that the agent increases the amount of an NME family member whose multimerization state is the biologically inactive state or decreases the relative amount of an NME family member whose multimerization state is the biologically active state.
41. The method according to paragraph 40, wherein the NME family member whose multimerization state is the biologically active state is NME7.
42. The method according to paragraph 40, wherein NME family member whose multimerization state is the biologically inactive state NME1, NME2 or NME8.

## Claims

1. An *in vitro* method for inducing growth in and/or maintaining an undifferentiated state in stem cells, the method comprising contacting the cells with an effective amount of an exogenous nucleoside diphosphate protein kinase 7 (NME7) comprising the A and B domains and lacking the M leader sequence.

2. The method of claim 1, wherein the stem cells are embryonic or induced pluripotent cells or progenitor cells.

3. The method of claim 2, wherein the cells are pluripotent cells and maintenance of an undifferentiated state is maintenance of pluripotency, wherein the pluripotent cell comprises a modified gene or protein expression profile, wherein the modified gene or protein expression profile comprises (1) an increased expression of a naive or pluripotency gene selected from OCT4, SOX2, NANOG, KLF4, KLF2, Tra 1-60, Tra 1-81, SSEA4, and REX-1, and (2) a decreased expression of a primed gene selected from LHX2, OTX2, XIST, and FOXA2 as compared to a corresponding expression profile of the pluripotent cell absent said contacting.

4. The method of claim 3, wherein the method provides a greater increase in the expression of the naive or pluripotency gene as compared to a corresponding increase achieved by basic fibroblast growth factor (bFGF).

5. The method of claim 1 wherein induction of growth of pluripotent cells provides a greater plurality of the pluripotent cells.

6. The method of claim 5, wherein the method provides the greater plurality of the pluripotent cells at a rate that is greater than a corresponding rate achieved with a nucleoside diphosphate protein kinase 1 (NME1) dimer.

7. The method of any one of claims 1-6, wherein the NME7 is a polypeptide or a polynucleotide encoding the polypeptide.

8. The method of any one of claims 1-7, wherein the NME7 is in a monomeric form.

9. The method of any one of claims 1-8, wherein the stem cells are human cells.

10. The method of any one of claims 2-9, wherein the pluripotent cells are **characterized by** one or more naive or pluripotency genes selected from KLF4, KLF2, SOX2, NANOG, LIN28 and OCT4.

11. The method of any one of claims 2-9, wherein the pluripotent cells are **characterized by** one or more naive or pluripotency genes selected from KLF4, KLF2, NANOG and OCT4.

12. The method of any one of claims 2-11, further comprising contacting the pluripotent cell with an inhibitor of nucleoside diphosphate protein kinase 1 (NME1), an inhibitor of nucleoside diphosphate protein kinase 2 (NME2), or an inhibitor of nucleoside diphosphate protein kinase 6 (NME6) to provide a decreased expression of NME1, a decreased expression of NME2, or a decreased expression of NME6 in the plurality of pluripotent cells as compared to a corresponding expression profile of the plurality of pluripotent cells absent said contacting.

13. An *in vitro* method for cell differentiation, the method comprising contacting a plurality of pluripotent cells with an effective amount of an exogenous inhibitor of nucleoside diphosphate protein kinase 7 (NME7) to provide a decreased expression of NME7 in the plurality of pluripotent cells as compared to a corresponding expression profile of the plurality of pluripotent cells absent said contacting, optionally wherein said inhibitor is an siRNA.

14. The method of claim 13, wherein the method provides a modified gene or protein expression profile in the plurality of pluripotent cells, wherein the modified gene or protein expression profile comprises (1) a decreased expression of a naive or pluripotency gene selected from OCT4, SOX2, NANOG, KLF4, KLF2, Tra 1-60, Tra 1-81, SSEA4, and REX-1, and (2) an increased expression of a primed gene selected from LHX2, OTX2, XIST, and FOXA2 as compared to a corresponding expression profile of the pluripotent cell absent said contacting.

15. The method of claim 13 or 14, further comprising contacting the plurality of pluripotent cells with an amount of an NME family member whose multimerization state is a biologically inactive state.
